# EUROPÄISCHE PATENTSCHRIFT

(11) **EP 2 654 429 B1**
(45) Veröffentlichungstag und Bekanntmachung des Hinweises auf die Patenterteilung: **25.07.2018**
(21) Anmeldenummer: 11758380.7
(22) Anmeldetag: 22.08.2011
(51) Int. Cl.: A01N 59/16, A61L 24/00, A61L 24/06, A61L 27/34, A61L 27/54

(54) **VERFAHREN ZUR HERSTELLUNG EINER SILBERNANOPARTIKELHALTIGEN DISPERSION SOWIE VERWENDUNG EINER SILBERNANOPARTIKELHALTIGEN MISCHUNG ALS BESCHICHTUNGSMITTEL**
PROCESS FOR PREPARING A SILVER NANOPARTICLES CONTAINING DISPERSION AND USE OF SILVER NANOPARTICLES CONTAINING MIXTURE AS COATING
PROCEDE DE PREPARATION D'UNE DISPERSION CONTENANT DES NANOPARTICULES D'ARGENT ET UTILISATION COMME REVETEMENT D'UNE COMPOSITION CONTENANT DES NANOPARTICULES D'ARGENT

(30) Priorität: 21.12.2010 WO PCT/DE2010/075165
(43) Veröffentlichungstag der Anmeldung: 30.10.2013
(73) Patentinhaber: aap Implantate AG, 12099 Berlin (DE)
(72) Erfinder: NUSKO, Robert, 93109 Wiesent (DE); MAIER, Georg, 93177 Altenthann (DE); DINGELDEIN, Elvira, 63933 Mönchberg (DE); WOLFSTÄDTER, Marco, 63939 Wörth/Main (DE)
(74) Vertreter: Blumbach · Zinngrebe Patent- und Rechtsanwälte
(86) Internationale Anmeldenummer: PCT/EP2011/004211
(87) Internationale Veröffentlichungsnummer: WO 2012/084072

(56) Entgegenhaltungen:
- WO-A1-2005/115151
- DE-A1-102006 056 284
- US-A1- 2007 003 603
- US-A1- 2008 181 931
- DATABASE WPI Week 200875, Derwent Publications Ltd., London, GB; AN 2008-M70940 & JP 2008 231489 A (DAIICHI KOGYO SEIYAKU CO LTD) 02 Oktober 2008

## Beschreibung

### Technisches Gebiet

Die Erfindung betrifft ein Beschichtungsmaterial mit einer silbernanopartikelhaltigen, dispersen Formulierung sowie Verfahren zu deren Herstellung und deren Verwendung, insbesondere als Beschichtungsmittel.

### Stand der Technik

Die biozide Wirkung von Silber ist bekannt. Insbesondere im medizinischen Bereich bei Implantaten wird zunehmend versucht, die Verwendung von Antibiotika zu reduzieren oder ganz auf die Verwendung von Antibiotika zu verzichten. Hierbei stellt Silber eine wirkungsvolle Alternative dar.

Problematisch hat sich bis lang erwiesen, dass zugesetzte Silberpartikel, insbesondere bei Knochenzement keine hinreichende Wirkung erzielen. Man vermutet, dass dies mit der in der Regel zu kleinen spezifischen Oberfläche des eingesetzten Materials zu tun hat.

Bei Knochenzement handelt es sich in der Regel um ein Material, was aufgrund einer Polymersationsreaktion aushärtet. In der Praxis bekannt ist beispielsweise Methylmethacrylat basierter Knochenzement. Dieser besteht in der Regel aus zwei Komponenten, nämlich einer Flüssig- und einer Festkomponente. Die Festkomponente kann ein größtenteils auspolymerisiertes Perlypolymerisat umfassen sowie ein Polymersationsstarter und weitere Komponenten, über die beispielsweise die Reaktionsgeschwindigkeit eingestellt wird. Die Monomerkomponente umfasst ein Monomer oder ein Prepolymer, über welches nach Vermischen der Flüssigkomponente mit der Festkomponente eine Polymersationsreaktion in Gang gesetzt wird, aufgrund der sich die zunächst pastöse Masse in einen Feststoff aushärtet. Knochenzement wird beispielsweise für den Einsatz von Endoprothesen, für die Herstellung von Spacern, bei mehrteiligen Prothesen und für Vertebro- und Kyphoplastie verwendet. Je nach gewünschtem Einsatzzweck können Knochenzemente mit unterschiedlichen Festigkeitseigenschaften und Aushärtungseigenschaften bereit gestellt werden.

Zur Bereitstellung einer antibiotischen Wirkung ist es bekannt ein Antibiotikum, wie beispielsweise Gentamicin, beizugeben.

Auch bei Knochenzement und polymerbasierbaren Beschichtungsmaterialien wäre es wünschenswert, zusätzlich oder als Alternative Silber zur Erzielung einer antimikrobiellen Wirkung zuzusetzen.

Aufgrund der besonderen Eigenschaften, insbesondere der größeren spezifischen Oberfläche wäre es insbesondere wünschenswert, nanopartikuläres Silber zuzusetzen. Ein Zuschlag von nanopartikulärem Silber zur festen Komponente scheitert in der Regel bereits daran, dass eine Bereitstellung von nanopartikulärem Silber in festem Zustand kaum möglich wäre, da dieses agglomeriert.

Auch der Zusatz von Silber in der Flüssigphase ist schwierig, da es zum einen zu Agglomerationseffekten kommt, und des Weiteren ist es bislang nicht gelungen, eine hinreichend stabile Dispersion mit nanopartikulärem Silber bereit zu stellen, welche auch in unpolaren oder in wenig polaren Flüssigkeiten wie Methylmethacrylat dispergiert bleibt.

Einer allgemeinen Definition folgend ist "Nanopartikel" eine Bezeichnung für Partikel, die eine Größe im Bereich kleiner als 100 nm aufweisen. Die Verwendung der Vorsilbe "Nano" stellt somit, entsprechend der offiziellen Definition nach ISO TC 229, eine Abgrenzung zu Partikel im Sub-Mikrometer Bereich (> 100 nm) dar,. Generell ist davon auszugehen, dass Stoffe, die als Nanomaterial bezeichnet werden, veränderte chemische und physikalische Eigenschaften besitzen. Bei Nanometallen zeigen z.B. Gold und Silber andere Farben als die entsprechenden Metalle, nämlich Rot bzw. Gelb.

Darüber hinaus ist es wissenschaftlich belegt, dass Nanopartikel eines Stoffes eine erhöhte Oberflächenenergie besitzen. Je kleiner die Partikel sind desto höher ist deren Oberflächenenergie. In der Konsequenz sind Nanopartikel generell als instabil zu betrachten, da sie aufgrund ihrer hohen Oberflächenenergie leicht zu neuen Verbindungen bzw. größeren, stabileren Aggregaten reagieren. Für das Beispiel der Nanometalle bedeutet dies, dass selbst die Partikel edler Metalle schnell mit Luftsauerstoff oxidieren sobald die Größe der Partikel im Bereich von Nanometern liegt.

Technologisch nutzbare Nanopartikel erhält man also nur, wenn deren Oberflächen chemisch oder physikalisch geschützt und damit stabilisiert sind. Als "technologisch nutzbar" können Nanopartikel bezeichnet werden, die von der Herstellung über die Verarbeitung bis hin zur Anwendung unverändert ihre ursprüngliche Partikelgröße behalten oder konservieren.

Möglichkeiten der Stabilisierung von Nanopartikeln in Dispersionen sind aus dem Stand der Technik bekannt. Es existieren drei wesentliche Verfahrenswege zur Herstellung von metallischen Nanopartikeln. In einem ersten Verfahren werden die Nanopartikel zur Stabilisierung auf Feststoffen geträgert. Die Feststoffe liegen dabei immer in einer stabilen Größe im Mikrometerbereich vor. Nachteilig bei der Verwendung der auf diese Weise hergestellten Produkte ist einerseits der Verlust der Nanoskaligkeit und andereseits die hohe Füllstofffracht. Der verwendete Füllstoff, der als Basis für die Entstehung der Metallnanopartikel dient, weist Korngrößen im Bereich von Mikrometern auf und ist z.B. für die Herstellung von dünnen Strukturen oder Fasern gänzlich ungeeignet. In der Praxis beträgt zudem der Gewichtsanteil des Füllstoffes ein Vielfaches des Nanometallanteiles.

Bei flammenpyrolytischen Verfahrenswegen fällt das Cluster bestehend aus Mikro- und Nanopartikeln als Feststoff an, der zur Weiterverwendung erst aufwändig redispergiert werden muss, was aufgrund von Lagerungseinflüssen oft nicht mehr vollständig möglich ist. Darüber hinaus kann die Verteilung der Nanopartikel niemals optimal erfolgen, da sie höchstens so gut sein kann wie die Verteilung der Mikropartikel, auf denen sie abgeschieden sind.

Ein zweiter Verfahrensweg besteht in der Synthese von Metallnanopartikel durch Stabilisierung mittels Polymeren wie Polyvinylpyrrolidon im Polyolprozess, der vielfach in der Literatur als Standardverfahren beschrieben ist. Allerdings werden hier nur geringe Metallnanopartikelkonzentrationen erreicht (Bereich kleiner 0.1 Gew.-% Silber).

Die dritte Variante zur Erzeugung von Metallnanopartikel ist ein PVD Verfahren (Physical Vapor Deposition), bei dem das zugrunde liegende Metall verdampft wird. Zur Stabilisierung der so erzeugten Nanopartikel werden wiederum Polymere bzw. Silikone verwendet. Die Erzeugung von Metalldampf ist ein sehr energieaufwändiger Prozess, der evakuierte Prozesskammern erfordert. Diese Herstellungsverfahren sind damit unökonomisch. Zudem bereiten die verwendeten Polymere und Silikone bei der Weiterverarbeitung erhebliche prozesstechnische Probleme, da eine Redispergierung oft unmöglich ist.

Daneben ist aus der DE 10 2006 056 284 A1 die Herstellung einer antimikrobiell wirkenden wässrigen Dispersion durch Mischen einer wässrigen Dispersion nanoscaliger Teilchen, die wenigstens ein antimikrobiell wirkendes Metall enthalten, mit einer wässrigen Dispersion eines Polymerisations-, Polykondensations- oder Polyadditionsproduktes bekannt. Die Herstellung der Silber-Nanopartikel erfolgt durch eine chemische Reduktion in Wasser. Zur Stabilisierung der Silber-Nanopartikel wird Natriumchlorid verwendet

Ein Nachteil, den alle Herstellungsvarianten zeigen, ist die schlechte Verarbeitbarkeit der Metallnanopartikel in Polymerschmelzen, wie etwa bei der Additivierung von thermoplastischen Polymeren. Feststoffe lassen sich ohne vorherige Dispergierung nicht homogen einarbeiten.

Es besteht daher weiterhin ein Bedarf an stabilen Dispersionen von Silbernanopartikeln mit antimikrobiellen Eigenschaften.

Das Dokument US 2008/181931 A1 zeigt das Zufügen eines Stabilisators und das Bilden von Nanopartikeln in situ. Das Dokument US 2007/003603 A1 zeigt die Verwendung einer wässrigen oder nicht wässrigen Dispersion als Beschichtungsmittel für Implantate. Das Dokument JP 2008-231489 A zeigt die Herstellung von nanoskaligen Metallpartikeln in Mizellen.

### Darstellung der Erfindung

Der Erfindung, wie sie in den Ansprüchen gekennzeichnet ist, liegt die Aufgabe zugrunde, stabile Dispersionen von Silbernanopartikeln bereitzustellen, die insbesondere in Knochenzement oder als antibakterielle Beschichtung für Implantate und medizinische Geräte eingesetzt aber auch werden können.

Insbesondere wird die Dispersion in Polymer-basierten Knochenzementen und Beschichtungsmaterialien verwendet.

Die Aufgabe der Erfindung wird bereits durch ein Verfahren zur Herstellung einer silbernanopartikelhaltigen Dispersion, durch eine silbernanopartikelhaltige Dispersion sowie durch die Verwendung einer silbernanopartikelhaltigen Mischung nach einem der unabhängigen Ansprüche gelöst. Weitere vorteilhafte Details, Aspekte und Ausgestaltungen der vorliegenden Erfindung ergeben sich aus den abhängigen Ansprüchen, der Beschreibung, den Beispielen und den Figuren.

Die Erfindung betrifft zum Einen ein Verfahren zur Herstellung einer silbernanopartikelhaltigen Dispersion. Diese Dispersion soll insbesondere zur Herstellung von Knochenzement oder für ein Beschichtungsmittel, insbesondere für Implantate und medizinische Instrumente verwendet werden. Weiter ist eine Verwendung als antibakterielles Trägermaterial sowohl im medizinischen Bereich als auch für Bekleidungs- und Gebrauchsgegenstände vorgesehen.

Gemäß der Erfindung wird ein Silbersalz und ein Stabilisator bereitgestellt.

Weiter wird ein Reduktionsmittel und ein organisches polymerisierbares Lösungsmittel bereitgestellt. Als organisches polymerisierbares Lösungsmittel sind insbesondere Polymere oder Prepolymere vorgesehen, welche sodann beispielsweise als Beschichtungsmittel verwendet werden können oder als Komponente eines Knochenzements.

Aus Silbersalz, Stabilisator und Reduktionsmittel wird eine Lösung hergestellt.

Nach Herstellen der Lösung werden eine Base sowie ein anorganisches Salz zugegeben.

Mit Zugabe der Base fallen Nanopartikel aus, welche dispergieren.

Über das anorganische Salz wird das in der Lösung verbleibende Wasser hydratisiert. Es bildet sich nunmehr eine wässrige Phase, wohingegen die Silbernanopartikel aufgrund des zugesetzten Stabilisators überwiegend in dem organischen polymerisierbaren Lösungsmittel verbleiben. Die wässrige Phase kann nunmehr abgetrennt, beispielsweise abdekandiert werden, so dass ein polymerisierbares organisches Lösungsmittel mit Silbernanopartikeln zurückbleibt.

Durch die Erfindung kann somit ein im Wesentlichen wasserfreies, silbernanopartikelhaltiges Monomer oder Prepolymer bereitgestellt werden, beispielsweise ein Acrylat, insbesondere Methylmethacrylat oder Buthylacrylat.

Es versteht sich, dass ein gewisser Anteil Wasser in dem organischen Lösungsmittel zurückbleiben kann, da geringe Mengen Wasser beispielsweise in Methylmethacrylat löslich sind.

Es bleibt aber eine im Wesentlichen organische polymerisierbare Lösung zurück, welche beispielsweise als Beschichtungsmaterial oder als Komponente von einem polymerisierbaren Material, insbesondere von Knochenzement verwendet werden kann.

Die Erfindung betrifft des Weiteren eine silbernanopartikelhaltige Dispersion, welche insbesondere als Knochenzement, antibakterielles Trägermaterial oder Beschichtungsmittel verwendet wird.

Die silbernanopartikelhaltige Dispersion umfasst Silbernanopartikel, zumindest einen Stabilisator und zumindest ein Netz- und Dispergieradditiv, wobei die Silbernanopartikel in einem flüssigen Monomer, Prepolymer oder Polymer dispergiert sind.

Der Erfindung liegt die Erkenntnis zugrunde, dass über einen Stabilisator sowie über ein weiteres Netz- und Dispergieraditiv es möglich ist, eine Dispersion in einer organischen Flüssigkeit, beispielsweise einem Acrylat mit Silbernanopartikeln bereitzustellen, welche auch über längere Zeit stabil ist.

Der Stabilisator ist vorzugsweise ausgewählt aus der Gruppe bestehend aus Polyoxyethylen-mono-alkylsäureester, Polyoxypropylen-mono-alkylsäureester, Polyoxyethylen-di-alkylsäureester, Polyoxypropylen-di-alkylsäureester, Polyoxyethylen-tri-alkylsäureester, Polyosypropylen-tri-alkylsäureester und deren Gemische.

Als Netz- und Dispergieradditiv wird vorzugsweise ein nichtionisches Tensid, insbesondere ein siliziumorganisches Tensid verwendet.

Die Erfinder vermuten, dass Nanopartikel von dem Stabilisator und sodann als zweite Schicht von dem Netz-und Dispergiermittel umhüllt sind.

Während der Stabilisator insbesondere in einem ersten Herstellungsschritt dazu dient, dass Nanopartikel in einer wässrigen Lösung ausfallen und nicht agglomerieren, wird über das Netz- und Dispergieradditiv erreicht, dass die Nanopartikel auch in einer wenig polaren organischen Flüssigkeit dispergiert bleiben. Die silbernanopartikelhaltige Dispersion kann beispielsweise als Monomer, insbesondere zur Herstellung von Knochenzement, für eine Beschichtungslösung oder als Zuschlagstoff für Polymerwerkstoffe verwendet werden.

Als Polymer wird dabei insbesondere ein Acrylat oder eine Acrylatvorstufe, insbesondere Methylmethacrylat verwendet. In einer Ausführungsform wird eine silbernanopartikelhaltigen Mischung verwendet, umfassend Silbernanopartikel und zumindest einen Stabilisator ausgewählt aus der Gruppe bestehend aus Polyoxyethylen-mono-alkylsäureester, Polyoxypropylen-mono-alkylsäureester, Polyoxyethylen-di-alkylsäureester, Polyoxypropylen-di-alkylsäureester, Polyoxyethylen-tri-alkylsäureester und Polyoxypropylen-tri-alkylsäureester.

Diese Mischung, welche für den erfindungsgemäßen Knochenzement oder das Beschichtungsmittel verwendet wird, soll im Folgenden im Detail beschrieben werden.

Im vorliegenden Text beziehen sich sämtliche Angaben von Anteilen in Gew.-% auf das Gewicht der gesamten Formulierung als 100%-Basis.

Die verwendete Mischung enthält zumindest einen Stabilisator ausgewählt aus der Gruppe bestehend aus Polyoxyethylen-mono-alkylsäureester, Polyoxypropylen-mono-alkylsäureester, Polyoxyethylen-di-alkylsäureester, Polyoxypropylen-di-alkylsäureester, Polyoxyethylen-tri-alkylsäureester und Polyoxypropylen-tri-alkylsäureester. Bei diesen Stabilisatoren handelt es sich um Verbindungen mit grenzflächenaktiven Eigenschaften aus der Gruppe der nichtionischen Tenside, die bei Raumtemperatur in flüssiger Form vorliegen. Nichtionische Tenside im Sinne der Erfindung sind grenzflächenaktive chemische Komponenten, die ungeladene polare und unpolare Bereiche in einem Molekül aufweisen. Darüber hinaus weisen nichtionische Tenside keine dissoziierbaren funktionellen Gruppen auf.

Eine erfindungsgemäße silbernanopartikelhaltige Mischung enthält dispersionsstabilisierte Silbernanopartikel, die nicht zu größeren Agglomeraten aggregieren können, da die verwendeten Stabilisatoren im Temperaturbereich von 0-240 °C flüssig sind. Im Gegensatz dazu hält der Stand der Technik beispielsweise viele Silbernanopartikelprodukte bereit, die als trockene Pulver angeboten werden, die aber aufgrund ihrer Agglomerationsneigung während Transport und Lagerung zur Dispergierung in organischen Lösungsmitteln, wie Methylmethacrylat nur unter hohem mechanischen Energieeintrag und dann auch nur unvollständig redispergiert werden können.

Besonders bevorzugt zur Verwendung in der vorliegenden Erfindung sind Kombinationen aus grenzflächenaktiven Komponenten aus den vorgenannten Chemikalienklassen. Gemäß einer besonders bevorzugten Ausführungsform sind in der Mischung also zumindest zwei Stabilisatoren anwesend.

Bei Anwesenheit mehrerer Stabilisatoren kann es sich dabei um verschiedene Stabilisatoren einer der genannten Klassen chemischer Verbindungen handeln oder um Stabilisatoren verschiedener Verbindungsklassen. Bei einer Kombination von drei verschiedenen Stabilisatoren können also beispielsweise drei verschiedene Polyoxyethylen-mono-alkylsäureester eingesetzt werden, es können aber beispielsweise auch zwei verschiedene Polyoxyethylen-mono-alkylsäureester und ein Polyoxypropylen-mono-alkylsäureester oder beispielsweise ein Polyoxypropylen-di-alkylsäureester, ein Polyoxyethylen-tri-alkylsäureester und ein Polyoxypropylen-tri-alkylsäureester verwendet werden. Es ist jede beliebige Kombination dieser nichtionischen Tenside möglich.

Besonders bevorzugt besteht die Mischung von Stabilisatoren aus einer Kombination nichtionischer Tenside aus zwei unterschiedlichen der oben genannten Verbindungsklassen.

Gemäß einer besonders bevorzugten Ausführungsform ist der Stabilisator bzw. sind die Stabilisatoren ausgewählt aus der Gruppe bestehend aus Polyoxyethylen-SorbitanMonolaurat, Polyoxyethylen-Sorbitan-Monopalmitat, Polyoxyethylen-Sorbitan-Monostearat, Polyoxyethylen-Sorbitan-Monooleat, Polyoxyethylen-Sorbitan-Tristearat, Polyoxyethylen-Glyceryl-Trioleat, Polyoxyethylen-Glyceryl-Monolaurat, Polyoxyethylen-Glyceryl-Monooleat, Polyoxyethylen-Glyceryl-Monostearat, Polyoxyethylen-Glyceryl-Monoricinoleat, Rizinusöl, hydriertes Rizinusöl und Sojabohnenöl.

Die Stabilisatoren sind vielfach nicht unter ihrem Chemikaliennamen bekannt sondern unter ihrer jeweiligen Handelsbezeichnung. Im Rahmen der vorliegenden Erfindung bevorzugte Stabilisatoren sind Tween20 ™, Tween40 ™, Tween60 ™, Tween80 ™, Polysorbat ™, Tagat TO ™, Tagat TO V ™, Tagat L2 ™, Tagat S2 ™, Tagat R40 ™, Triton X 100 ™, Hydrogenated Castoroil ™, PEG 20 Glycerylstearat ™, PEG 20 Glyceryllaurat ™, PEG 40 Castoroil ™, PEG 25 Glyceryltrioleat ™, Newcol ™, Montane ™, Lonzest ™, Liposorb ™, Nonion ™, Kuplur ™, Ionet ™, Kemotan ™, Grillosan ™, Ethylan ™, Glycomul ™, Emsorb ™, Disponil ™, Amisol ™, Armotan ™, Sorbax ™, Sorbitan ™, Span ™ und Tego Pearl ™.

Diese Liste von Stabilisatoren ist nicht vollständig, da verschiedene Hersteller gleiche oder ähnliche Produkte unter anderen Namen vermarkten bzw. neue nichtionische Tenside der oben genannten Verbindungsklassen in der Zukunft synthetisiert werden und ebenfalls in einer erfindungsgemäßen Mischung verwendet werden können.

Sind zumindest zwei Stabilisatoren in der Mischung enthalten, so sind diese bevorzugt in einem Mengenverhältnis im Bereich von 1 : 1 bis 2 : 1 in der Mischung anwesend.

Da die in der Mischung enthaltenen nichtionischen Tenside als Stabilisatoren für das gebildete Nanometall wirken, besteht ein Mengenzusammenhang zwischen den Konzentrationen des Stabilisators und des Metalls. Gemäß einer weiteren bevorzugten Ausführungsform liegt das Mengenverhältnis Silbernanopartikel zu Stabilisator im Bereich von 10 : 2 bis zu 10 : 50, besonders bevorzugt im Bereich von 10 : 5 bis zu 10 : 10. Sind mehrere Stabilisatoren anwesend, so ist unter dem "Mengenverhältnis Metall zu Stabilisator" das "Mengenverhältnis Metall zur Summe der anwesenden Stabilisatoren" zu verstehen. Bei Verwendung der bevorzugten Mengenverhältnisse erhält man Mischungen, aus denen besonders stabile Dispersionen von Metallnanopartikeln hergestellt werden können, welche universell einsetzbar sind.
Bevorzugt weisen die Metallnanopartikel eine Partikelgröße von 1 bis 100 nm, besonders bevorzugt 1 bis 50 nm, insbesondere bevorzugt 1 bis 20 nm auf.

Die vorliegende Erfindung bezieht sich auch auf eine silbernanopartikelhaltige Formulierung umfassend eine Dispersion einer der oben beschriebenen metallnanopartikelhaltigen Mischungen. Die erfindungsgemäße Formulierung ist flüssig und enthält ansonsten keinerlei feste Nebenbestandteile, die die Möglichkeiten der weiteren Verwendung einschränken würden.

In der erfindungsgemäßen Mischung wie auch in der erfindungsgemäßen Formulierung sind eine oder mehrere grenzflächenaktive Komponenten als Stabilisatoren enthalten, die neben der Stabilisierung der Silbernanopartikel auch die Weiterverarbeitung (durch nochmalige Dispergierung, Emulgierung) in alle anderen Substrate ermöglichen. Die technologisch anspruchsvollste Weiterverarbeitung ist dabei die Prozessierung in thermoplastischen Kunststoffen. Die hierbei angewendeten Temperaturen reichen bis 300 °C. Bis zu dieser Temperatur ist es wünschenswert, dass die zur Additivierung verwendete Formulierung flüssig ist, was durch die Verwendung eines oder mehrere grenzflächenaktiver Komponenten, die bis zu einer Temperatur von kurzzeitig 300°C flüssig sind, erfüllt ist. Besonders bevorzugt sind in der Formulierung zumindest zwei Stabilisatoren anwesend. Bei Anwesenheit mehrerer Stabilisatoren kann es sich dabei um verschiedene Stabilisatoren einer der genannten Klassen chemischer Verbindungen handeln oder um Stabilisatoren verschiedener Verbindungsklassen. Bei einer Kombination von drei verschiedenen Stabilisatoren können also beispielsweise drei verschiedene Polyoxyethylen-mono-alkylsäureester eingesetzt werden, es können aber beispielsweise auch zwei verschiedene Polyoxyethylen-mono-alkylsäureester und ein Polyoxypropylen-mono-alkylsäureester oder beispielsweise ein Polyoxypropylen-di-alkylsäureester, ein Polyoxyethylen-tri-alkylsäureester und ein Polyoxypropylen-tri-alkylsäureester verwendet werden. Es ist jede beliebige Kombination dieser nichtionischen Tenside möglich.

Besonders bevorzugt besteht die Mischung von Stabilisatoren aus einer Kombination nichtionischer Tenside aus zwei unterschiedlichen der oben genannten Verbindungsklassen.

Da das zumindest eine nichtionische Tensid als Stabilisator für das gebildete Nanometall verwendet wird besteht ein Mengenzusammenhang zwischen den Konzentrationen des Stabilisators und des Silbers. Die erfindungsgemäße Formulierung zur Verwendung beispielsweise in Methylmethacrylat weist ein Mengenverhältnis Silber zu Stabilisator im Bereich von 10 : 2 bis 10 : 50 auf. Bevorzugt beträgt das Mengenverhältnis Metall zu Stabilisator von 10 : 5 bis 10 : 20, besonders bevorzugt von 10 : 6 bis 10 : 10. Sind mehrere Stabilisatoren anwesend, so ist unter dem "Mengenverhältnis Metall zu Stabilisator" das "Mengenverhältnis Metall zur Summe der anwesenden Stabilisatoren" zu verstehen. Bei Verwendung der bevorzugten Mengenverhältnisse erhält man besonders stabile Dispersionen von Silbernanopartikeln Bevorzugt sind mehr als zwei Stabilisatoren in der erfindungsgemäßen Formulierung anwesend. In diesem Fall liegt der Gehalt eines ersten Stabilisators im Bereich von 30 bis 90 Gew.-%, bevorzugt zwischen 40 bis 60 Gew.-%, besonders bevorzugt zwischen 45 bis 55 Gew.-%. Der verbleibende Gewichtsanteil bis 100 Prozent teilt sich unter die weiteren in Kombination verwendeten erfindungsgemäßen Stabilisatoren auf, die sich ihrerseits in Mengenanteilen von 0 bis 100 Gew.-% aufteilen. Die hier genannten Gew.-%-Angaben beziehen sich somit in Abweichung zu sonstigen Angaben auf das Gesamtgewicht an Stabilisatoren als 100%-Basis.

Besonders bevorzugt sind in der Formulierung ein oder mehrere Stabilisatoren ausgewählt aus der Gruppe bestehend aus Tagat TO V ™, Tween20 ™, Tween80 ™ und Tagat L2 ™ anwesend. Unter Verwendung dieser Stabilisatoren werden besonders stabile und universell einsetzbare Dispersionen erhalten.

Gemäß einer ganz besonders bevorzugten Ausführungsform der vorliegenden Erfindung ist in der Formulierung als Stabilisator ein Gemisch von Tagat TO V ™ und Tween20 ™ anwesend. Insbesondere bevorzugt sind Formulierungen, in denen das Mengenverhältnis Tagat TO V ™ zu Tween20 ™ im Bereich von 1 : 2 bis zu 2 : 1 liegt und ganz besonders bevorzugt sind Formulierungen, in denen das Mengenverhältnis Tagat TO V ™ zu Tween20 ™ ungefähr 1 : 1 beträgt.

Bezüglich der Partikelgröße sei nochmals auf die eingangs formulierte Definition verwiesen, wonach die Silbernanopartikel eine Partikelgröße von weniger als 100 nm aufweisen. In der erfindungsgemäßen Formulierung liegen die Silbernanopartikel in einer Partikelgröße von 1 bis 100 nm, bevorzugt 1 bis 50 nm, besonders bevorzugt 1 bis 20 nm vor. Die Morphologie der Silbernanoteilchen kann dabei Formen von Dreiecken, Kuben, Sphären, Stäben oder Plättchen aufweisen.

Bevorzugt enthält die Formulierung stabile, nanoskalige Metall-Partikel in einer Konzentration von 0,5 bis 60 Gew.-%, wobei das Mengenverhältnis Silbernanopartikel zu Stabilisator im Bereich von 10 : 2 bis zu 10 : 50, bevorzugt im Bereich von 10 : 5 bis zu 10 : 10 liegt. In den bevorzugten Bereichen erhält man besonders stabile Dispersionen von Silbernanopartikeln, die universell eingesetzt werden können.

Besonders bevorzugt sind die Silbernanopartikel in einem Anteil von 1 bis 40 Gew.-%, bevorzugt in einem Anteil von 5 bis 30 Gew.-% in der Formulierung enthalten. Ein organisches Lösungsmittel wird zur Herstellung der Dispersion eingesetzt. Es handelt sich dann also um eine Dispersion einer der oben näher beschriebenen silbernanopartikelhaltigen Mischungen in einem organischen Lösungsmittel. Ganz besonders bevorzugt handelt es sich bei dem organischen Lösungsmittel um Methylmethacrylat.

Die vorliegende Erfindung umfasst auch ein Verfahren zur Herstellung der oben beschriebenen metallnanopartikelhaltigen, dispersen Formulierungen umfassend die Schritte Bereitstellen eines Metallsalzes, Bereitstellen zumindest eines Stabilisators ausgewählt aus der Gruppe bestehend aus Polyoxyethylen-mono-alkylsäureester, Polyoxypropylen-mono-alkylsäureester, Polyoxyethylen-di-alkylsäureester, Polyoxypropylen-di-alkylsäureester, Polyoxyethylen-tri-alkylsäureester, Polyoxypropylen-tri-alkylsäureester, Bereitstellen eines Reduktionsmittels, Bereitstellen eines Lösungsmittels, Herstellen einer Lösung von Metallsalz, Stabilisator und Reduktionsmittel, Zugabe einer Base zu der Lösung, wobei die Zugabe der Base kontinuierlich über einen Zeitraum von 5 bis 48 h derart erfolgt, dass der pH- Wert der Formulierung zwischen 0 und 6 liegt.

Durch das erfindungsgemäße Herstellungsverfahren wird eine Formulierung mit einer sehr engen Verteilung der Partikelgrößen der Nanopartikel erhalten. Als Lösungsmittel wird ein organisches Lösungsmittel verwendet.

Bevorzugt erfolgt die Zugabe der Base kontinuierlich über einen Zeitraum von 9 bis 30 h. Auf diese Weise wird eine Formulierung mit einer besonders engen Verteilung der Partikelgrößen der Nanopartikel erhalten.

Das erfindungsgemäße Herstellungsverfahren ist ein reduktiver chemischer Prozess. Demnach werden die Silberpartikel durch chemische Reduktion aus ihren Salzen hergestellt. Generell kann zur Herstellung der erfindungsgemäßen Silbernanopartikel jedes beliebige chemische oder physikalische Reduktionsmittel verwendet werden. Unter physikalischen Reduktionsmitteln ist hier Temperaturerhöhung oder Bestrahlung mit Licht zu verstehen. Vorteilhaft ist die Verwendung eines chemischen Reduktionsmittels, da hier Stoffumsätze von 100 Prozent und sehr hohe Reaktionsgeschwindigkeiten erzielt werden können.

Überraschenderweise hat sich gezeigt, dass bei Anwesenheit zumindest eines Stabilisators aus der Gruppe Polyoxyethylen-mono-alkylsäureester, Polyoxypropylen-mono-alkylsäureester, Polyoxyethylen-di-alkylsäureester, Polyoxypropylen-di-alkylsäureester, Polyoxyethylen-tri-alkylsäureester und Polyoxypropylen-tri-alkylsäureester sehr starke Reduktionsmittel verwendet werden können, was zu einer erhöhten Reaktionsgeschwindigkeit führt, ohne gleichzeitige Gefahr der Bildung eines größeren Anteils großer, unerwünschter Silberpartikel.

Unter den chemischen Reduktionsmitteln sind solche bevorzugt, die keine in der Reaktionsmischung verbleibenden Reaktionsnebenprodukte, wie etwa die entsprechende oxidierte Form des jeweiligen Reduktionsmittel, erzeugen, welche die Qualität der silbernanopartikelhaltigen, dispersen, wässrigen Formulierung mindern würden. Gemäß einer bevorzugten Ausführungsform wird daher ein Reduktionsmittel eingesetzt, das mit den Metallionen des Metallsalzes unter Bildung von elementarem Metall und ansonsten überwiegend gasförmigen Reaktionsprodukten reagiert.

Besonders bevorzugt werden Reduktionsmittel, die in ihrer oxidierten Form als gasförmiger Stoff die Reaktionslösung verlassen können, wie etwa Hydrazinhydrat. Selbstverständlich können die erfindungsgemäßen Silbernanopartikel ebenso mit jedem beliebigen anderen Reduktionsmittel erhalten werden.

Die reduktive Herstellung von Metallen kann als ein Paar von Redox-Gleichungen formuliert werden. Die erste Teilgleichung ist die Reduktionsgleichung, nach der das Metallkation aus dem Metallsalz zum Elementmetall reduziert wird. Die zweite Teilgleichung beschreibt den entsprechenden oxidativen Vorgang für die Oxidation des Reduktionsmittel zum korrespondierenden Oxidationsprodukt, das idealerweise in gasförmigem Zustand die Reaktionslösung verlässt. Allen Reduktionsmitteln ist gemeinsam, dass je übertragenem Elektron ein Proton entsteht. Dieses Proton trägt dazu bei, dass der pH-Wert der gesamten Reaktionslösung sehr stark fällt. Die Abnahme des pH-Wertes ist dafür verantwortlich, dass die Reaktion unerwünschterweise zum Erliegen kommt. Deshalb muss eine Lauge zugegeben werden, um die entstehenden Protonen, die die Gesamtreaktion bremsen, abzufangen.

Überraschenderweise wurde gefunden, dass die Art der Lauge, die Konzentration der Lauge und die Geschwindigkeit, mit der die Lauge zugegeben wird, entscheidend ist für die Verteilung der Partikelgrößen der Nanopartikel in der hergestellten Formulierung.

Bevorzugt wird als Base Ammoniak, Kaliumhydrogencarbonat oder Natriumhydroxid eingesetzt. Bei Verwendung dieser Basen werden besonders stabile Dispersionen mit einer engen Verteilung der Partikelgrößen der Nanopartikel erhalten.

Die zugegebene Menge an Lauge ist dabei so zu bemessen, dass nach Abschluss der Reaktion eine pH neutrale Dispersion erhalten wird. Der pH-Wert liegt dann zwischen pH 5 und pH 9.

Laugen bzw. Protonen-Akzeptoren sind definiert durch ihre pK_{b} Werte. Der pK_{b} Wert ist der negative dekadische Logarithmus der Protonenkonzentration im Gleichgewicht und ist somit ein Maß für die Stärke der Base.

Zur Herstellung der erfindungsgemäßen Formulierung sind Basen geeignet, die einen pK_{b} Wert im Bereich von -2 bis 10,5, bevorzugt 1,5 bis 9,1, besonders bevorzugt im Bereich 3,5 bis 7,5 aufweisen.

Neben der Basenstärke ist zudem die Zugabegeschwindigkeit in die Reaktionslösung zur Herstellung der erfindungsgemäßen Formulierung entscheidend. Erfolgt die Zugabe zu schnell, so verschiebt sich das Partikelgrößen-Spektrum hin zu größeren Partikeln, die im Extremfall im Mikrometerbereich liegen. Erfolgt dagegen die Zugabe zu langsam, so werden keine Stoffausbeuten größer 90 % erhalten, da bereits gebildetes Nanometall katalytisch den Abbau von Reduktionsmitteln hervorruft und damit kein Reaktionspartner mehr zur Erzeugung von Silbernanopartikeln vorliegt.

Experimente haben gezeigt, dass die Zugabegeschwindigkeit der Lauge bei einer Ansatzgröße von 50 kg im Bereich von 9 bis 30 Stunden liegen sollte, um die erfindungsgemäße hohe Qualität an Silbernanopartikeln zu erreichen. Bei entsprechend geringeren Ansatzgrößen verringert sich analog auch die Zeit der Laugenzugabe. Nach oben kann die Zugabezeit nicht beliebig erweitert werden, da der katalytische Abbau des Reduktionsmittels durch bereits entstandenes Nanometall spätestens nach 48 h die Gesamtausbeute merklich beeinträchtigt.

Die Zugabegeschwindigkeit der Lauge erfolgt so, dass der pH Wert der Dispersion stets zwischen 0 und 6 liegt. Ein höherer pH Wert führt zu einer zu schnellen Reaktion und damit zu unkontrolliertem Partikelwachstum. Ein zu niedriger pH Wert führt dazu, dass die Reaktion zum Erliegen kommt und damit kein Nanometall mehr gebildet wird.

Die erfindungsgemäße Formulierung kann in einer Vielzahl von Anwendungen zum Einsatz kommen, wobei bei den verschiedensten Verwendungen deutlich vorteilhafte Eigenschaften erreicht werden. Die Metallnanopartikel der erfindungsgemäßen Formulierung bzw. die Metallnanopartikel einer nach dem erfindungsgemäßen Verfahren hergestellten Formulierung können insbesondere zur Erzielung antimikrobieller Aktivität in verschiedene Substrate eingearbeitet werden.

Die vorliegende Erfindung bezieht sich außerdem auf ein Verfahren zur Herstellung zur Herstellung von Knochenzement oder eines Beschichtungsmittels für Implantate oder medizinische Instrumente mit einer der oben näher beschriebenen silbernanopartikelhaltigen Mischungen, wobei zu einer der oben näher beschriebenen silbernanopartikelhaltigen Formulierungen ein anorganisches Salz zugegeben wird oder nach Durchführung eines der oben näher beschriebenen Verfahren zur Herstellung einer silbernanopartikelhaltigen Formulierung ein anorganisches Salz zugegeben wird, wobei das anorganische Salz zumindest ein Element der vierten oder der fünften Hauptgruppe des Periodensystems der Elemente als Bestandteil des Anions umfasst.

Die vorliegende Erfindung umfasst ein
- Verfahren zur Herstellung einer silbernanopartikelhaltigen Mischung umfassend Silbernanopartikel und zumindest einen Stabilisator ausgewählt aus der Gruppe bestehend aus Polyoxyethylen-mono-alkylsäureester, Polyoxypropylen-mono-alkylsäureester, Polyoxyethylen-di-alkylsäureester, Polyoxypropylen-di-alkylsäureester, Polyoxyethylen-tri-alkylsäureester und Polyoxypropylen-tri-alkylsäureester, umfassend die Schritte
   - Bereitstellen eines Silbersalzes,
   - Bereitstellen zumindest eines Stabilisators ausgewählt aus der Gruppe bestehend aus Polyoxyethylen-mono-alkylsäureester, Polyoxypropylen-mono-alkylsäureester, Polyoxyethylen-di-alkylsäureester, Polyoxypropylen-di-alkylsäureester, Polyoxyethylen-tri-alkylsäureester, Polyoxypropylen-tri-alkylsäureester und deren Gemische,
   - Bereitstellen eines Reduktionsmittels,
   - Bereitstellen eines Lösungsmittels,
   - Herstellen einer Lösung von Silbersalz, Stabilisator und Reduktionsmittel,
   - Zugabe einer Base zu der Lösung, wobei die Zugabe der Base kontinuierlich über einen Zeitraum von 5 bis 48 h derart erfolgt, dass der pH- Wert der Formulierung zwischen 0 und 6 liegt,
   - Zugabe eines anorganischen Salzes, wobei das anorganische Salz zumindest ein Element der vierten oder der fünften Hauptgruppe des Periodensystems der Elemente als Bestandteil des Anions umfasst.

Eine erfindungsgemäße nanopartikelhaltige Mischung kann also durch Zugabe eines anorganischen Salzes aus einer entsprechenden nanopartikelhaltigen Formulierung gewonnen werden.

Insbesondere bei der Herstellung der nanopartikelhaltigen Mischung aus einer nanopartikelhaltigen Formulierung, die durch Reduktion eines Silbersalzes in Lösung gewonnen wurde, ist die Zugabe eines anorganischen Salzes mit ganz besonderen Vorteilen verbunden. Es wurde nämlich überraschenderweise gefunden, dass sich durch Zugabe anorganischer Salze die durch Zugabe einer Base zu der Lösung von Silbersalz, Stabilisator und Reduktionsmittel erhaltene Dispersion in zwei chemische Phasen 1 und 2 trennt. Phase 1 enthält dabei die Silbernanopartikel in dem verwendeten flüssigen Stabilisatorengemisch. Das Lösungsmittel, die anorganischen Salze und das Nebenprodukt Ammoniumnitrat befinden sich in der über der Phase 1 stehenden Phase 2. Die beiden Phasen lassen sich nunmehr auf einfache Weise voneinander separieren, indem die obere Phase 2 abdekantiert wird. Zurück bleibt die Phase 1, die nur mehr aus den Silber-Nanopartikeln und den flüssigen Stabilisatoren besteht.

Die in Form einer Dispersion vorliegenden Silbernanopartikel, die in der Regel Partikelgrößen von 1-20 nm aufweisen und chemisch stabilisiert sind, werden auf diese Weise von dem in der Dispersion enthaltenen Nebenprodukt Ammoniumnitrat und dem verwendeten Lösungsmittel abgetrennt. Die erfindungsgemäßen silbernanopartikulären Formulierungen können dadurch zusätzlichen Applikationsfeldern zugänglich gemacht werden.

Zur näheren Charakterisierung geeigneter Salze müssen deren kationische und anionische Bestandteile gesondert betrachtet werden. Salze bestehen generell aus mindestens einem Kation und mindestens einem Anion. Eine unerwünschte Wechselwirkung von Kationen mit der stabilisierten metallnanopartikelhaltigen Formulierung ist nicht zu erwarten, da das verwendete Silber entweder als ungeladenes Metall oder als positiv geladenes Kation vorliegt.

Die Auswahl der Anionen ist zudem dadurch eingeschränkt, dass ungewünschte Wechselwirkungen mit vorhandenen Silber-Kationen vermieden werden müssen. Damit scheiden alle Anionen aus, die mit dem eingesetzten Silber schwerlösliche Verbindungen bilden, also z.B. Halogenide, Chalkogenide und deren Sauerstoffverbindungen.

Besonders bevorzugt umfasst das anorganische Salz daher zumindest ein Element der fünften Hauptgruppe des Periodensystems der Elemente als Bestandteil des Anions, wobei insbesondere bevorzugt das anorganische Salz Stickstoff als Bestandteil des Anions umfasst.

Besonders bevorzugt erfolgt anschließend ein Abdekantieren der sich nach Zugabe des anorganischen Salzes bildenden Phase von der im wesentlichen aus Silbernanopartikel und Stabilisatoren bestehenden silbernanopartikelhaltigen Mischung.

Die vorliegende Erfindung bezieht sich außerdem auf ein Verfahren zur Herstellung einer der oben näher beschriebenen silbernanopartikelhaltigen Formulierungen umfassend die Schritte Bereitstellen einer der oben näher beschriebenen silbernanopartikelhaltigen Mischungen, Bereitstellen eines Lösungsmittels, Zugabe der silbernanopartikelhaltigen Mischung zu dem Lösungsmittel. Die vorliegende Erfindung umfasst also ein Verfahren zur Herstellung einer silbernanopartikelhaltigen Formulierung umfassend eine Dispersion einer silbernanopartikelhaltigen Mischung umfassend Silbernanopartikel und zumindest einen Stabilisator ausgewählt aus der Gruppe bestehend aus Polyoxyethylen-mono-alkylsäureester, Polyoxypropylen-mono-alkylsäureester, Polyoxyethylen-di-alkylsäureester, Polyoxypropylen-di-alkylsäureester, Polyoxyethylen-tri-alkylsäureester und Polyoxypropylen-tri-alkylsäureester umfassend die Schritte Bereitstellen einer silbernanopartikelhaltigen Mischung umfassend Silbernanopartikel und zumindest einen Stabilisator ausgewählt aus der Gruppe bestehend aus Polyoxyethylen-mono-alkylsäureester, Polyoxypropylen-mono-alkylsäureester, Polyoxyethylen-di-alkylsäureester, Polyoxypropylen-di-alkylsäureester, Polyoxyethylen-tri-alkylsäureester und Polyoxypropylen-tri-alkylsäureester, Bereitstellen eines Lösungsmittels, Zugabe der silbernanopartikelhaltigen Mischung zu dem Lösungsmittel.

Die erfindungsgemäßen chemisch stabilisierten Silbernanopartikel können durch das Lösungsmittel benetzt bzw. gelöst werden, ohne die zur Stabilisierung nötige Stabilisatorhülle zu verlieren. Bei dem Lösungsmittel handelt es sich um ein organisches Lösungsmittel. Verwendet werden können alle organischen, protischen, aprotischen, polaren und unpolaren Verbindungen bzw. deren Mischungen.

Gemäß einer besonders bevorzugten Ausführungsform der vorliegenden Erfindung wird zusätzlich zumindest ein Netz-und Dispergieradditiv zugegeben. Die Netz- und Dispergierhilfsmittel gewährleisten die Benetzung bzw. Lösung der Silbernanopartikel durch das verwendete Lösungsmittel.

Geeignete chemische Verbindungen, die als Netz- und Dispergieradditive verwendet werden können sind Alkylphenolethoxylate, aminofunktionelle Polyester, phosphorhaltige Substanzen wie beispielsweise organisch modifizierte Phosphate, Phosphonate, Polyphosphorverbindungen und Alkylphosphonate, oder eine Mischung dieser Verbindungen.

Besonders bevorzugt handelt es sich bei dem Netz- und Dispergieradditiv um ein organisch modifiziertes Phosphat, ein Phosphonat, eine Polyphosphorverbindung, ein Alkylphosphonat, eine Phosphorverbindung mit gemischten organischen Liganden, ein Oligomer oder ein Polymer mit phosphathaltigen Liganden.

Solche Netz- und Dispergieradditive werden von den Firmen Evonic, BYK Chemie und Ciba Geigy angeboten.

Die chemisch stabilisierten Silbernanopartikel mit einer bevorzugten Partikelgröße von 1-20 nm können also unter Zuhilfenahme von Netz-und Dispergieradditiven u.a. in organischen Lösungsmitteln, insbesondere in Methylmethacrylat, eingearbeitet werden. Diese Einarbeitung kann mit einfachsten Rühr- oder Mischtechniken erfolgen, da eine Redispergierung der Metallnanopartikel durch die Verwendung der erfindungsgemäßen Stabilisatoren nicht erforderlich ist. Auf diese Weise werden stabile Dispersionen von beispielsweise Silbernanopartikeln mit einer Partikelgröße von bevorzugt kleiner 20 nm in organischen Lösungsmitteln, bevorzugt in Methylmethacrylat, in einer Konzentration von 5.000 mg/kg bis 50.000 mg/kg Silbergehalt, erhalten.

Die Erfindung betrifft insbesondere einen Knochenzement, ein antibakterielles Trägermaterial oder ein Beschichtungsmittel, insbesondere ein acrylatbasiertes Beschichtungsmittel, welches mit dem vorstehend beschriebenen Verfahren herstellbar ist.

Weiter betrifft die Erfindung einen Knochenzement, ein antibakterielles Trägermaterial oder ein Beschichtungsmittel für Implantate oder medizinische Geräte, welches Silbernanopartikel umfasst.

Das Beschichtungsmittel ist insbesondere ein Flüssigbeschichtungsmittel, beispielsweise ein Acrylat oder Silicon. Das Beschichtungsmittel kann beispielsweise durch Eintauchen (Dip-Coaten) aufgebracht werden.

Gemäß der Erfindung sind die Nanopartikel mit zumindest einem ersten und zum einem zweiten Stabilisator umhüllt und in einem Polymer dispergiert.

Unter einem Polymer wird jede Form von Prepolymer sowie auch eine im Wesentlichen noch nicht umgesetzte Monomerlösung, welche beispielsweise überwiegend Methylmethacrylat umfasst, verstanden.

Die Erfinder haben herausgefunden, dass durch die Verwendung zweier verschiedener Stabilisatoren, insbesondere durch die Verwendung zweier Emulgatoren, es möglich ist, eine stabile Dispersion bereit zu stellen, welche auch in unpolaren Flüssigkeiten erhalten bleibt.

Als Ausgangsmaterial eignet sich insbesondere die vorstehend beschriebene Formulierung, bei welcher man sich vorstellt, dass diese bereits Silbernanopartikel mit einer Hülle aus zumindest einem Stabilisator umfasst.

Aber auch bei dieser Formulierung ist nicht immer sichergestellt, dass es nicht zu Ausfäll- und Agglomerationserscheinungen kommt.

Die Erfinder haben aber herausgefunden, dass durch die Auswahl eines zweiten Stabilisators, von dem man vermutet, dass er sich wie eine zweite Hülle um den ersten Stabilisator legt, es möglich ist, eine stabile Dispersion in einer unpolaren Flüssigkeit bereit zu stellen.

Hierfür wird insbesondere ein nichtionisches Tensid, insbesondere ein siliciumorganisches Tensid verwendet. Ein solches Tensid ist beispielsweise unter dem Handelsnamen Tego DISPERS 655 erhältlich.

Insbesondere werden der Mischung, also beispielsweise der Monomerkomponente eines Knochenzements oder der Beschichtungslösung mindestens 0,1, vorzugsweise mindestens 0,2 % des zweiten Stabilisators zugesetzt. Das Ziel ist dabei, die Menge an zusätzlichen chemischen Stoffen möglichst gering zu halten.

Die Erfinder haben herausgefunden, dass eine Menge von unter 1 vorzugsweise unter 0,2 % ausreicht, um Nanopartikel mit einer mittleren Partikelgröße zwischen 5 und 50, vorzugsweise zwischen 10 und 20 nm zu stabilisieren.

So lässt sich eine Dispersion bereitstellen, bei welcher zumindest 90, vorzugsweise zumindest 99 % der Silbernanopartikel kleiner als 50, vorzugsweise kleiner als 20 nm sind.

Vorzugsweise weisen die Nanopartikel eine im Wesentlichen sphärische Form auf, wobei unter einer sphärischen Form im Sinne der Erfindung eine Form verstanden wird, bei der sich Länge, Breite und Höhe der Partikel weniger als 20 % voneinander unterscheiden, es mithin keine z.B. nadelförmigen Partikel sind.

Insbesondere mit einem Anteil an Silbernanopartikeln zwischen 0,5 und 5 vorzugsweise zwischen 1 und 3 Gewichtsprozent in dem Polymer lassen sich polymerbasierte Beschichtungen oder Knochenzemente bereitstellen, welche eine antimikrobe Wirkung aufweisen und bei denen der Einsatz an Antibiotika zumindest reduziert oder sogar ganz auf den Einsatz von Antibiotika verzichtet werden kann.

Die vorliegende Erfindung umfasst auch die Verwendung der erfindungsgemäßen Formulierung zur Oberflächenbehandlung von Implantaten und medizinischen Geräten. Die mit dieser Art der Verwendung erzielten besonderen Vorteile werden in den nachfolgenden Beispielen näher erläutert.

Die vorliegende Erfindung umfasst insbesondere die Verwendung der erfindungsgemäßen Formulierung zur Herstellung antimikrobieller Oberflächen. Die mit dieser Art der Verwendung erzielten besonderen Vorteile werden in den nachfolgenden Beispielen näher erläutert.

Die vorliegende Erfindung umfasst auch die Verwendung der erfindungsgemäßen Formulierung in Silikonen als Beschichtungsmaterial. Die mit dieser Art der Verwendung erzielten besonderen Vorteile werden in den nachfolgenden Beispielen näher erläutert.

Die vorliegende Erfindung umfasst auch die Verwendung der erfindungsgemäßen Formulierung in thermoplastischen Kunststoffen, bevorzugt in Polypropylen. Die mit dieser Art der Verwendung erzielten besonderen Vorteile werden in den nachfolgenden Beispielen näher erläutert.

Die vorliegende Erfindung umfasst auch die Verwendung der erfindungsgemäßen Formulierung in Duroplasten, bevorzugt die Verwendung zur Herstellung von PMMA Knochenzement. Die mit dieser Art der Verwendung erzielten besonderen Vorteile werden in den nachfolgenden Beispielen näher erläutert.

Die vorliegende Erfindung umfasst auch die Verwendung der erfindungsgemäßen Formulierung zur Herstellung von PMMA Beschichtungen. Die mit dieser Art der Verwendung erzielten besonderen Vorteile werden in den nachfolgenden Beispielen näher erläutert.

### Kurze Beschreibung der Zeichnungen

Zur Illustration der Erfindung und zur Verdeutlichung ihrer Vorzüge werden nachfolgend Ausführungsbeispiele angegeben.

Diese Ausführungsbeispiele sollen im Zusammenhang mit den Zeichnungen näher erläutert werden. Es versteht sich von selbst, dass diese Angaben die Erfindung nicht beschränken sollen. Es zeigen
- Fig. 1: ein UVvis-Spektrum einer 5.000-fach verdünnten wässrigen Lösung einer erfindungsgemäßen Formulierung;
- Fig. 2: Gemessene Partikelgrößen und berechnete Kurve der Scanning Electron Microscope (SEM)-Datensätze.
- Fig. 3: Transmission Electron Microscope (TEM) Analyse der Silber-Nanopartikel.
- Fig. 4: eine transmissionselektronenmikroskopische Aufnahme (TEM) einer 5.000-fach verdünnten wässrigen Lösung einer erfindungsgemäßen Formulierung;
- Fig. 6: eine transmissionselektronenmikroskopische Aufnahme (TEM) eines beschichteten Vlies-/Folienlaminats;
- Fig. 7: die Kinetik der Abtötung von auf das beschichtete Vlies der Figur 6 aufgebrachten Bakterien (*E.coli.*)*;*
- Fig. 8: eine transmissionselektronenmikroskopische Aufnahme (TEM) eines Polyester Masterbatches mit 6500 mg/kg Silber;
- Fig.: Microfaserstränge aus PET/PA mit 200 mg/kg
- Fig. 10: Elutionsverhalten und antimikrobielle Aktivität verschiedener Polyester Mikrofasern.

Folgende Beispiele sind nicht erfindungsgemäss.

### Beispiel 1:

### Formulierung von Nanosilber mit Hydrazinhydrat, Ammoniak, Tagat TO V™ und Tween20

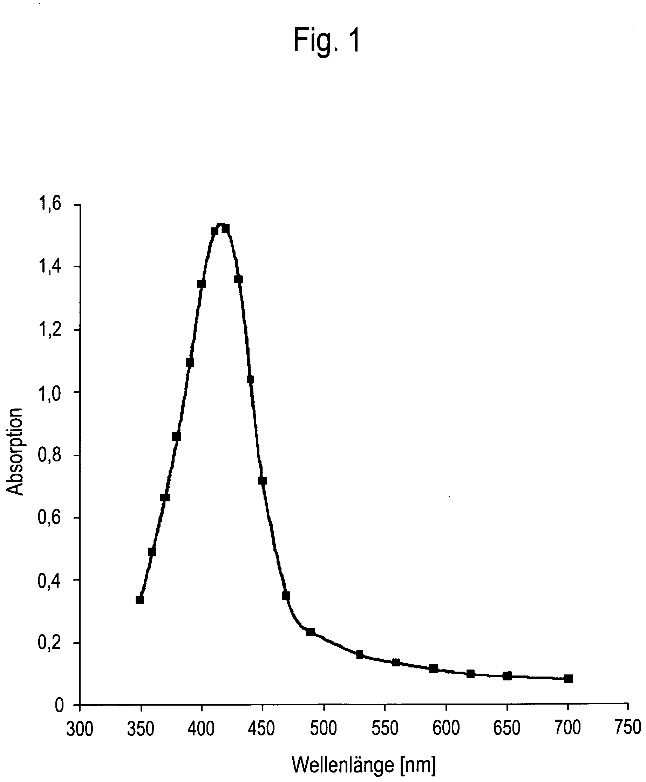

Vorgelegt werden 7.000 g Silbernitrat, 1.760 g Tagat TO V ™, 1.760 g Tween20 ™ und 512 g Hydrazinhydrat in 28.439 g entionisiertem Wasser. Die Lösung wird für 3 Stunden gerührt. Dann werden 5.000 g Ammoniak-Lösung (14 %-ig) über einen Zeitraum von 24 Stunden kontinuierlich zugetropft. Die Reaktion ist nach erfolgter Zugabe abgeschlossen und liefert eine Dispersion mit einem Silbergehalt von 10.0 Gew.-%. Die Partikelgröße und Verteilung wird mittels eines UVvis Spektrums (Figur 1) ermittelt. Im Ergebnis wird eine 10 prozentige Nanosilberdispersion erhalten mit einer Nanosilber-Partikelgröße von 1-30 nm.

Das Absorptionsspektrum wird an einer 5.000-fach verdünnten wässrigen Lösung durchgeführt, die 20 ppm Nanosilber enthält, klar und tiefgelb gefärbt ist. Das UVvis Spektrum wird im Wellenlängenbereich von 750 bis 350 nm aufgenommen. Die gemessenen Absorptionswerte liefern einen Peak mit einem Maximum bei 410-420 nm und einer Peak-Halbwertsbreite von rund 80 nm.

Die Dispergiereigenschaften der erhaltenen 10 prozentigen Dispersion sind sowohl in polaren als auch in unpolaren Lösungmittel hervorragend, das heißt es wird ohne weiteren chemischen Aufwand (Dispergierhilfen) oder mechanischen Aufwand (Ultraschall, Ultraturax etc.) eine absolut klare Lösung, die lediglich eine durch den Plasmoneffekt des Silbers verursachte Färbung aufweist, erhalten.

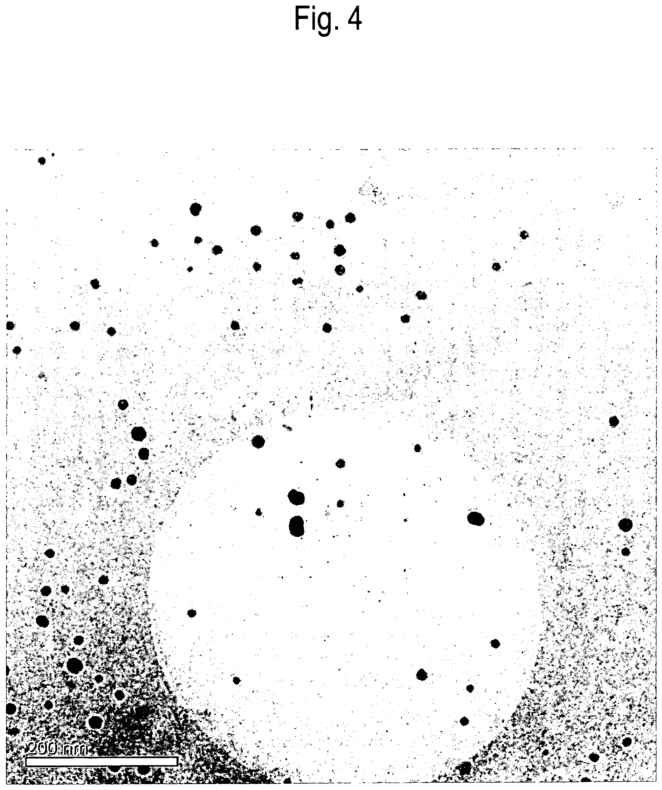

Figur 4 zeigt eine Transmissionselektronenmikroskopische Aufnahme (TEM) der verdünnten Dispersion aus Beispiel 1. Die in der Figur 4 sichtbaren dunklen Bereiche entsprechen den Nano-Silber-Partikeln, die eine Partikelgröße von 1 - 30 nm aufweisen.

### Beispiel 2:

### Formulierung von Nanosilber mit Hydrazinhydrat, Ammoniak und Tagat TO V ™

Vorgelegt werden 7.000 g Silbernitrat, 3.520 g Tagat TO V ™ und 1.331 g Hydrazinsulfat in 27.620 g entionisiertem Wasser. Die Lösung wird für 3 Stunden gerührt. Dann werden 5.000 g Ammoniak-Lösung (14 %-ig) über einen Zeitraum von 24 Stunden kontinuierlich zugetropft. Die Reaktion ist nach erfolgter Zugabe abgeschlossen und liefert eine Dispersion mit einem Silbergehalt von 10.0 Gew.-%. Die Partikelgröße und Verteilung wird mittels eines UVvis -Spektrums ermittelt. Im Ergebnis wird eine 10 prozentige Nanosilberdispersion erhalten mit einer Nanosilber-Partikelgröße von 1-30 nm.

### Beispiel 3:

### Formulierung von Nanosilber mit Hydrazinsulfat, Kaliumhydrogencarbonat, Tagat TO V ™ und Tween80 ™

Vorgelegt werden 7.000 g Silbernitrat, 2.360 g Tagat TO V ™, 1.160 g Tween80 ™ und 1.331 g Hydrazinsulfat in 27.620 g entionisiertem Wasser. Die Lösung wird für 3 Stunden gerührt. Dann werden 5.000 g Kaliumhydrogencarbonat-Lösung (1.900 g KHCO₃) über einen Zeitraum von 30 Stunden kontinuierlich zugetropft. Die Reaktion ist nach erfolgter Zugabe abgeschlossen und liefert eine Dispersion mit einem Silbergehalt von 10.0 Gew.-%. Die Partikelgröße und Verteilung wird mittels eines UVvis-Spektrums (Figur 1) ermittelt. Im Ergebnis wird eine 10 prozentige Nanosilberdispersion erhalten mit einer Nanosilber-Partikelgröße von 1-30 nm.

### Beispiel 4:

### Formulierung von Nanosilber mit Glucose, Natriumhydroxid, Tagat L2 ™ und Tween20 ™

Vorgelegt werden 7.000 g Silbernitrat, 2.360 g Tagat L2 ™, 1.160 g Tween20 ™ und 3.708 g Glucose in 25.243 g entionisiertem Wasser. Die Lösung wird für 3 Stunden gerührt. Dann werden 5.000 g Natriumhydroxid-Lösung (760 g NaOH) über einen Zeitraum von 30 Stunden kontinuierlich zugetropft. Die Reaktion ist nach erfolgter Zugabe abgeschlossen und liefert eine Dispersion mit einem Silbergehalt von 10.0 Gew.-%. Die Partikelgröße und Verteilung wird mittels eines UVvis Spektrums ermittelt. Im Ergebnis wird eine 10 prozentige Nanosilberdispersion erhalten mit einer Nanosilber-Partikelgröße von 1-30 nm.

### Beispiel 5:

### Formulierung von Nanokupfer mit Hydrazinhydrat, Ammoniak, Tagat TO V ™ und Tween20 ™

Vorgelegt werden 10.000 g Kupfer(II)nitrat, 1.760 g Tagat TO V ™, 1.760 g Tween20 ™ und 1.090 g Hydrazinhydrat in 14.260 g entionisiertem Wasser. Die Lösung wird für 3 Stunden gerührt. Dann werden 5.000 g Ammoniak-Lösung (14 %-ig) über einen Zeitraum von 24 Stunden kontinuierlich zugetropft. Die Reaktion ist nach erfolgter Zugabe abgeschlossen und liefert eine Dispersion mit einem Kupfergehalt von 10.0 Gew.-%.

### Beispiel 6:

### Weiterverarbeitung der Dispersion aus Beispiel 1 zur Herstellung einer flüssigen, wasser- und salzfreien Formulierung mit Silbernanopartikel

Die in Beispiel 1 erhaltene Silbernanopartikel enthaltende wässrige Dispersion ist mit dem Nebenprodukt Ammoniumnitrat verunreinigt. Die Silbernanopartikel weisen Partikelgrößen von 1-20 nm auf und sind chemisch stabilisiert. Der Gehalt an Silber beträgt 25 Gew.-%. Der Gehalt an Wasser beträgt 366 g und der Gehalt an Ammoniumnitrat 185 g. Von dieser Dispersion werden 1.000 g in ein Becherglas gegeben und unter Rühren auf 45°C geheizt. Die Trennung der Dispersion in zwei Phasen wird durch Zugabe von 78 g Kaliumnitrat eingeleitet. Nach erfolgter Zugabe und dem vollständigen Auflösen des Kaliumnitrats wird die Heizung entfernt und der Rührer ausgeschaltet. Die Trennung der Phasen kann nach Abkühlung beobachtet werden. Die obere, klare, wässrige Phase 1 wird vollständig abdekantiert. Die zurückbleibende Phase 2 besitzt eine dunkelbraune Farbe mit sirupartiger Fließeigenschaft und einem Gewicht von 449 g. Die stabile Dispersion ist nun bereit für die Einarbeitung in ein beliebiges organisches Lösungsmittel, insbesondere Methylmethacrylat.

Eine Analyse der wässrigen Phase 1 ergibt einen Salzgehalt von 263 g und einen Wassergehalt von 366 g.

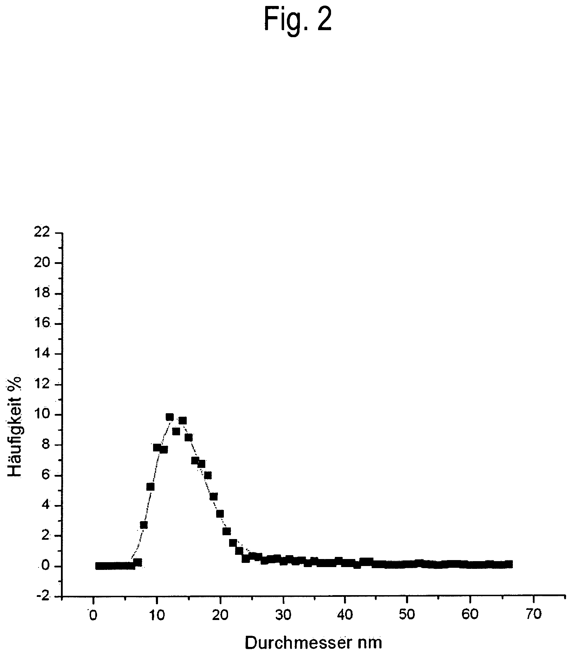

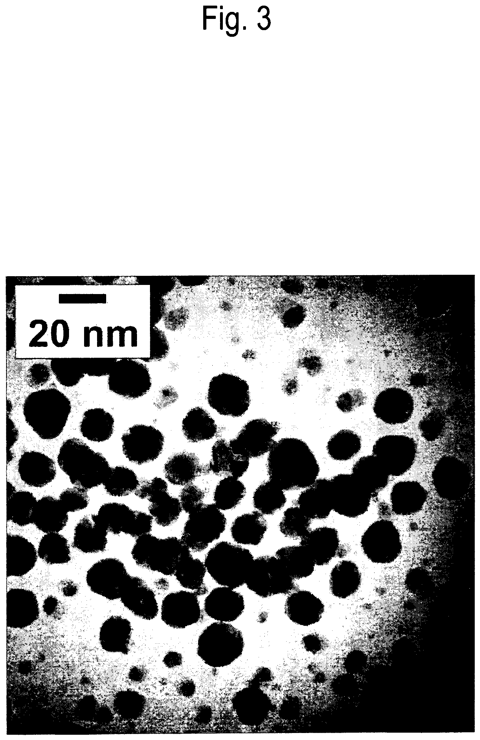

Die Analyse der silberhaltigen Phase 2 ergibt folgende Daten:
a) Die Analyse des Gesamt-Silbergehaltes durch Veraschung bei 800°C ergibt 250 g Silber. Bezogen auf die Gesamtformulierung entspricht dies einem Silbergehalt von 56 Gew-%.
b) Die Analyse der Partikelgrößenverteilung des erhaltenen Silbers ist in Figur 1 wiedergegeben. Die Bestimmung erfolgt durch Messung des UVvis-Absorptionsspektrums im Wellenlängenbereich von 700 nm - 350 nm. Das Peakmaximum bei 415 nm entspricht einer Partikelgröße von 10 nm. Die Peakhalbwertsbreite von maximal 80 nm ist ein Maß für die enge Partikelgrößenverteilung. Die Korrelation mit den SEM (Scanning Electron Microscope) und den TEM (Transmission Electron Microscope) Analysen, wie in Figur 2 und Figur 3 dargestellt, erlaubt die Angabe der Partikelgrößenverteilung von D 100 < 20 nm (100 % der Partikeldurchmesser sind kleiner als 20 nm).

### Beispiel 7:

### Weiterverarbeitung der Dispersion aus Beispiel 1 zur Herstellung einer flüssigen, wasser- und salzfreien Formulierung mit Silbernanopartikel

Die in Beispiel 1 erhaltene Silbernanopartikel enthaltende wässrige Dispersion ist mit dem Nebenprodukt Ammoniumnitrat verunreinigt. Die Silbernanopartikel weisen Partikelgrößen von 1-20 nm auf und sind chemisch stabilisiert. Der Gehalt an Silber beträgt 10 Gew.-%. Der Gehalt an Wasser beträgt 746 g und der Gehalt an Ammoniumnitrat 74 g. Die Trennung der Dispersion in zwei Phasen wird durch Zugabe von 202 g Kaliumnitrat eingeleitet. Nach erfolgter Zugabe und dem vollständigen Auflösen des Kaliumnitrats wird die Heizung entfernt und der Rührer ausgeschaltet. Die Trennung der Phasen kann nach Abkühlung beobachtet werden. Die obere, klare, wässrige Phase 1 wird vollständig abdekantiert. Die zurückbleibende Phase 2 besitzt eine dunkelbraune Farbe mit sirupartiger Fließeigenschaft und einem Gewicht von 180 g. Die stabile Dispersion ist nun bereit für die Einarbeitung in ein beliebiges organisches Lösungsmittel, insbesondere Methylmethacrylat.

Eine Analyse der wässrigen Phase 1 ergibt einen Salzgehalt von 276 g und einen Wassergehalt von 746 g.
Die Analyse der silberhaltigen Phase 2 ergibt folgende Daten:
a) Die Analyse des Gesamt-Silbergehaltes durch Veraschung bei 800°C ergibt 100 g Silber. Bezogen auf die Gesamtformulierung entspricht dies einem Silbergehalt von 55 Gew-%.
b) Die Analyse der Partikelgrößenverteilung des erhaltenen Silbers ist in Figur 1 wiedergegeben. Die Bestimmung erfolgt durch Messung des UVvis-Absorptionsspektrums im Wellenlängenbereich von 700 nm - 350 nm. Das Peakmaximum bei 415 nm entspricht einer Partikelgröße von 10 nm. Die Peakhalbwertsbreite von maximal 80 nm ist ein Maß für die enge Partikelgrößenverteilung. Die Korrelation mit den SEM (Scanning Electron Microscope) und den TEM (Transmission Electron Microscope) Analysen, wie in Figur 2 und Figur 3 dargestellt, erlaubt die Angabe der Partikelgrößenverteilung von D 100 < 20 nm (100 % der Partikeldurchmesser sind kleiner als 20 nm).

### Beispiel 8:

### Verwendung der erfindungsgemäßen Formulierung zur Oberflächenbehandlung von Holz

Die erfindungsgemäße Dispersion aus Beispiel 1 wird in einer Konzentration von 100 mg/kg (bezogen auf den Silbergehalt im fertigen Produkt) wie aus dem Stand der Technik bekannt in handelsübliches Leinöl eingearbeitet. Es wird eine stabile Nanopartikel-Dispersion erhalten, die sich zur Oberflächenbehandlung von Holz eignet.

Das mit dem additivierten Holzöl ausgerüstete Holz ist beständig gegen eine Vielzahl von Chemikalien und Wasser. Darüber hinaus sind die Holzoberflächen vor Besiedelung durch Mikroorganismen geschützt, d.h. dass aufgebrachte Mikroben auf den beschriebenen Oberflächen im Vergleich zu Oberflächen mit nicht additiviertem Holzöl schneller absterben.

### Beispiel 9:

### Verwendung der erfindungsgemäßen Formulierung zur Wachstumsförderung von Pflanzen

Die Dispersion aus Beispiel 1 wird in einer Konzentration von bevorzugt 1-100 pg/kg (bezogen auf den Silbergehalt im fertigen Produkt) in Wasser verteilt. Es wird eine stabile Nanopartikel-Dispersion erhalten, die zur Wachstumsförderung auf Pflanzen eingesetzt werden kann.

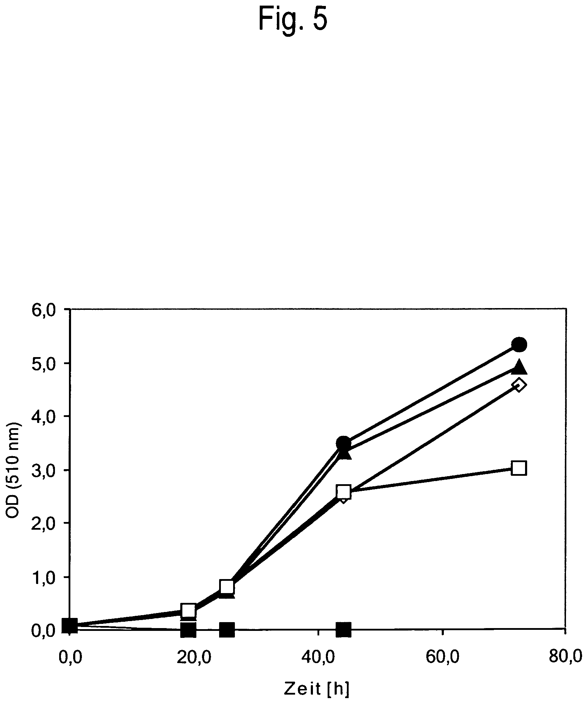

Die Wirkung der wässrigen Nanosilber-Dispersion auf das Pflanzenwachstum wurde mittels eines Algen-Kultivierungsversuch mit *Scenedesmus sp.* untersucht. Die Ergebnisse des Versuchs sind in Figur 5 wiedergegeben. In Figur 5 ist auf der Abszisse die Versuchsdauer nach erfolgter Nanosilber Zugabe aufgetragen. Auf der Ordinate ist die zur Biomassekonzentration (Algenkonzentration) proportionale Optische Dichte (OD) der Kultur bei 510 nm Wellenlänge aufgetragen. Es wurden Kulturen ohne Nanosilber Zugabe (□: 0 ppb nAg) und Kulturen mit unterschiedlichen Nanosilbergehalten (◊: 1 ppb, ●: 10 ppb, ▲: 100 ppb und ■: 1000 ppb) im Hinblick auf das Algenwachstum untersucht.

Wie aus Figur 5 zu entnehmen ist, wachsen die Algen ohne Nanosilber Zugabe in den ersten 42 Stunden der Kultivierung exponentiell bis zu einer OD von etwa 2,5. Danach kommt das Wachstum zum erliegen. Die OD der nicht additivierten Kultur stagniert bei etwa 3. Die Wachstumskurve der mit 1 pg/kg Nanosilber additivierten Kultur zeigt die ersten 42 Stunden des Versuchs einen der nicht additivierten Kultur analogen Verlauf. Während das Wachstum der nicht additivierten Kultur zwischen 42 Stunden und dem Versuchsende bei 75 Stunden fast zum Erliegen kommt, setzen die mit 1 µg/g Nanosilber additivierten Kulturen auch in diesem Zeitraum das Wachstum ungebremst fort und erreichen einen OD-Endwert von 4,7.

Die mit 10 µg/kg bzw. 100 µg/kg additivierten Algenkulturen zeigen im Vergleich mit der nicht additivierten Kultur bereits nach 25 Stunden ein deutlich erhöhtes Algenwachstum. Am besten wachsen die Algen in bei einer Nanosilber Zugabe von 10 ppb. Der erreichte Endwert nach 75 Stunden Versuchsdauer liegt bei einer OD von 5,4. Damit lässt sich in diesem Experiment durch eine Nanosilber Zugabe von 10 pg/kg zu *Scenedesmus sp.* eine Wachstumssteigerung von 80 % gegenüber einer nicht additivierten Algenkultur nach 75 Stunden Kultivierungsdauer nachweisen. Bei einer Zugabe von 1000 pg/kg Nanosilber zu der Kultur, wirkt das Silber toxisch auf die Algen. Die zu Beginn des Experiments zugegebenen Algen sterben rasch ab.

### Beispiel 10:

### Verwendung der erfindungsgemäßen Formulierung zur Herstellung antimirkobieller Oberflächen

Die Dispersion aus Beispiel 1 wird in einer Konzentration von bevorzugt 5-50 g/kg Silber mittels plasmaelektrolytischer Oxidation auf Metalloberflächen, wie z.B. Titan beschichtet. Es werden stark antimikrobiell aktive (R-Wert >3) Oberflächen erhalten.

### Beispiel 11:

### Verwendung der erfindungsgemäßen Formulierung zur Herstellung von Beschichtungen für Vlies-/Folienlaminate

Die Nanosilber Dispersion aus Beispiel wird in einer Konzentration von 150 mg/kg (bezogen auf den Silbergehalt in der Beschichtung) wie aus dem Stand der Technik bekannt in eine handelsübliche, zur Beschichtung von Folien bestimmte Fluorpolymerdisperion eingearbeitet. Es wird eine stabile, leicht gelb gefärbte Dispersion aus Nanosilber Partikeln und Fluorpolymerpartikeln erhalten. Diese Beschichtung wird auf ein PP-Spinnvlies/Folienlaminat aufgerakelt und thermisch getrocknet/fixiert/vernetzt.

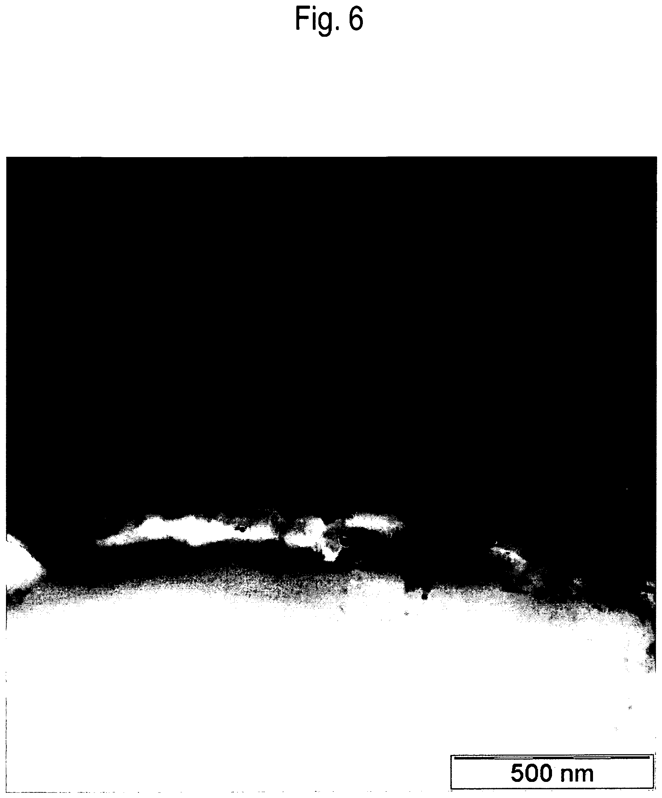

In Figur 6 ist eine TEM-Aufnahme der Beschichtung mit Nanosilber dargestellt. Die typischerweise 20 nm großen Nanosilberpartikel (schwarze Punkte in der TEM Aufnahme) sind nicht aggregiert und sehr gleichmäßig in der Polymerbeschichtung verteilt.

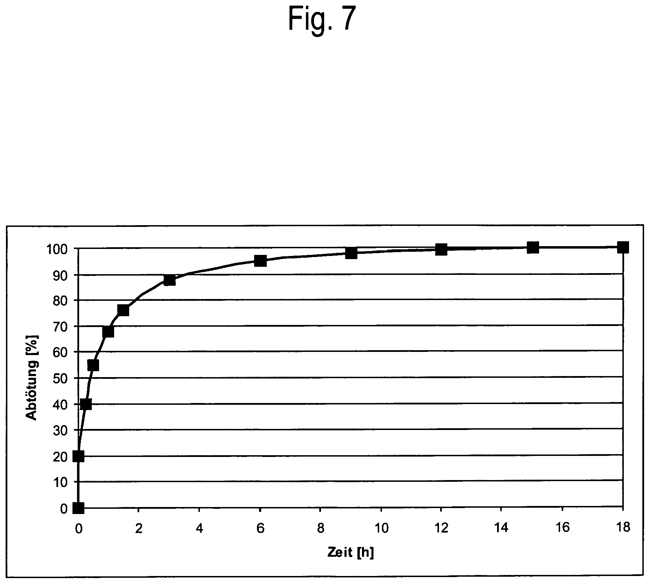

In Figur 7 ist die Kinetik der Abtötung von Bakterien (*E.coli*.), die auf das beschichtete Vlies-/Folienlaminat aufgebracht wurden dargestellt. Bereits nach 3,5 Stunden sind 90 % der aufgebrachten Keime abgetötet.

In der Tabelle 1 ist das Ergebnis eines Mikrobiologischen Tests nach JIS 2801 für das beschriebene Vlies-/Folienlaminat dargestellt. Bei dem Test werden jeweils 2X10⁵ Bakterien auf das mit Nanosilber beschichtete Vlies-/Folienlaminat, auf ein unbeschichtetes Vlies-/Folienlaminat und auf eine Standard Polystyroloberfläche gebracht. Nach einer Kultivierungszeit von 18 Stunden wird die Zahl der lebenden Keime bestimmt. Zusammengefasst lässt sich sagen, dass die Zahl der Keime auf dem Nanosilber beschichteten Vlies-/Folienlaminat im Vergleich zum Standard Polystyrol um 99,8 % reduziert werden, während das unbeschichtete Vlies-/Folienlaminat innerhalb der biologischen Schwankungsbreite keinerlei Keimreduktion aufweist. Damit gilt das Nanosilber beschichtete Vlies-/Folienlaminat als stark antimikrobiell wirksam.

In der Tabelle 2 ist das Ergebnis der Bestimmung der antimycotischen Aktivität des Nanosilber beschichteten Vlies-/Folienlaminats angegeben. Zusammengefasst lässt sich feststellen, dass das Nanosilber beschichtete Vlies-/Folienlaminat eine signifikante Aktivität gegen die 5 getesteten Pilze zeigt.

**Tabelle 1: Mikrobiologischer Test nach JIS 2801 für Nanosilber beschichtetes Vlies-/Folienlaminat mit 150 ppm**

| 1. Test Ergebnis | | | |
|---|---|---|---|
| Nach 0 h | Bakterienzahl Durchschnittswert [cfu] | | |
| Inoculum | 2,1x10⁵ | | |

| Nach 18 h | Bakterienzahl Durchschnittswert [cfu] | | F-Wert |
|---|---|---|---|
| interner Standard (Polystyrol) | 2,8x10⁶ | | 1,12 |

| 2-fach Bestimmung unabhängiger Proben | | | |
|---|---|---|---|
| Nach 18 h | Bakterienzahl Durchschnittswert [cfu] | % Reduktion ¹ | R-Wert |
| Vlies/Folie Laminat mit Nanosilber Beschichtung | 5,1x10³ | 99,8% | 2,74 |
| Vlies/Folie Laminat ohne Nanosilber Beschichtung | 1,8x10⁶ | 35,6% | 0,19 |

| 2. Methode: - | JIS Japanese Industrial Standard JIS Z 2801:2000 | | |
|---|---|---|---|
| | Antimicrobial products - Test for antimicrobial activity and efficacy. | | |
| | - Plate Count Method - | | |
| Test Bakterien: | *Escherichia Coli* K12 | | |
| | | | |
| Modifikation: | Probengröße:25 mm x 25 mm | | |
| | Berechnung: nur R-Wert | | |
| | Preinkubation C: LB Broth | | |
| | Preinkubation D: LB Broth | | |
| | Inoculations-Medium:1/500 verd. LB Broth (+ 0,13 % Tween 80) | | |
| | Incubation: 37°C | | |
| | Proben Präparation: UVC sterilisiert | | |

| | | | |
|---|---|---|---|
| 1) % Reduktion and R-Wert bezogen auf internen Standard | | | |

Silber in der Beschichtung.

**Tabelle 2: Bestimmung der antimycotischen Aktivität für Nanosilber beschichtetes Vlies-/Folienlaminat mit 150 ppm Silber in der Beschichtung.**

| 1. Test Ergebnis | | |
|---|---|---|
| Kontrolle | Pilzwachstum | Kat. |
| Wachstumskontrolle | stark | 6 |
| Kontrolle, Sterilität | nein | |

| Proben | Antimykotische Aktivität | Kat. |
|---|---|---|
| Baumwolle (interner Standard) | nein | 6 |
| Vlies-Folien-Laminat mit Beschichtung | Nanosilber-signifikant | 3 |
| | | |

| 2. Test Methode | SN 195921 - Textile Fabric - Determination of the Antimycotic Activity - modified | |
|---|---|---|
| | | |
| Test Stämme: | DSM 40464 - *Streptomyces abikoensis* | |
| | | |
| | DSM 9122 - *Scopulariopsis brevicaulis* | |
| | DSM 62413 - *Fusarium solani* | |
| | DSM 10640 - *Penicillium funiculosum* | |
| | DSM 2404 - *Aureobasidium pullulans* | |
| | | |
| Modifikation: | Probengröße: 30 mm Ø | |
| | Preinkubation: Potato Dextrose Agar | |
| | Suspensions-Medium: Potato Dextrose Bouillon | |
| | Proben-Inoculation: Sprühen | |
| | Inkubation: 25°C, Feuchtekammer | |
| | Inkubation-Zeit: 40 Tage | |
| | Proben-Sterilisation: UVC | |

### Beispiel 12:

### Verwendung der erfindungsgemäßen Formulierung zur Herstellung von Beschichtungen für textile Dekorstoffe

Die Nanosilber Dispersion aus Beispiel 1 wird in Konzentrationen von 100 mg/kg und 200 mg/kg (bezogen auf den Silbergehalt in der Beschichtung) mit den Mitteln des Standes der Technik in eine handelsübliche, zur Beschichtung von Textilien bestimmte Fluorpolymerdisperion eingearbeitet. Es wird eine stabile, leicht gelb gefärbte Dispersion aus Nanosilber Partikeln und Fluorpolymerpartikeln erhalten. Diese Beschichtung wird auf einen textilen Dekorstoff aufgebracht und thermisch getrocknet/fixiert/vernetzt.

In der Tabelle 3 sind die Ergebnisse mikrobiologischer Tests an Fußmattendekortextilien mit 2 unterschiedlichen hydrophoben, wässrigen Beschichtungen und jeweils zwei Nanosilberdosierungen nach JIS 1902 dargestellt. Die hydrophoben, wässrigen Beschichtungen AG4 und AG8 sind handelsübliche Fluorpolymercoatings. Bei der Beschichtung AG4 wird mittels eines Nanosilber Zusatzes von 100 mg/kg bzw. 200 mg/kg jeweils eine starke Keimreduktion gegenüber einem unbehandelten Textil beobachtet. Bei der Beschichtung AG8 ist der Nanosilber Zusatz von 100 mg/kg zur Hinderung des Keimwachstums nicht ausreichend. Ein Zusatz von 200 mg/kg hingegen bewirkt eine starke Keimreduktion gegenüber einem unbehandelten Textil.

**Tabelle 3: Mikrobiologische Tests nach JIS 1902 an Fußmattendekortextilien mit 2 unterschiedlichen hydrophoben, wässrigen Beschichtungen und jeweils zwei Silberdosierungen.**

| 1. Test Ergebnis | | | |
|---|---|---|---|
| Nach 0 h | Bakterienzahl Durchschnittswert [cfu] | | |
| Inoculum | 1,8x10⁵ | | |

| Nach 18 h | Bakterienzahl Durchschnittswert [cfu] | | F-Wert |
|---|---|---|---|
| Faser A0 (Blank) | 1,5x10⁷ | | 1,92 |

| 2-fach Bestimmung unabhängiger Proben | | | |
|---|---|---|---|
| Nach 18 h | Bakterienzahl Durchschnittswert [cfu] | % Reduktion 1 | R-Wert |
| Kontrolle: 080116-xhf01 Mesh fabric (Rita), DTY | 160/200F < 1,0x10² | 99,9993% | 5,18 |
| Fußmattendekormaterial Beschichtung AG4 100 ppm | 1,4x10⁴ | 99,9% | 3,03 |
| Fußmattendekormaterial Beschichtung AG4 200 ppm | 2,4x10⁵ | 98,4% | 1,79 |
| Fußmattendekormaterial Beschichtung AG8 100 ppm | 4,6x10⁷ | no | -0,49 |
| Fußmattendekormaterial Beschichtung AG8 200 ppm | 7,8x10³ | 99,9% | 3,29 |
| | | | |

| 2. Test Methode: | JIS Japanese Industrial Standard JIS L 1902:2002 "Testing for antibacterial activity and efficacy on textile products". | | |
|---|---|---|---|
| | - Plate Count Method - | | |
| | | | |
| Test Stamm: | *Escherichia Coli* K12 | | |
| | | | |
| Modifikation: | Einwaage: 0,4 g | | |
| | Berechnung: nur R-Wert | | |
| | Preinkubation C: LB Broth | | |
| | Preinkubation D: LB Broth | | |
| | Inoculations-Medium: Phosphate Buffered Saline mit 0.05% Tween 80 | | |
| | Inkubation: 37°C | | |

| | | | |
|---|---|---|---|
| 1)% Reduktion and R-Wert bezogen auf Blank | | | |

### Beispiel 13:

### Verwendung der erfindungsgemäßen Formulierung in Lacken und Klebstoffen

Die Nanosilber Dispersion aus Beispiels 1 wird in einer Konzentration von 270 mg/kg (bezogen auf den Silbergehalt im fertigen Produkt) wie aus dem Stande der Technik bekannt in handelsübliche, zur Lackierung von Holz (insbesondere zur Treppen- und Parkettversiegelung) geeignete Lacke eingearbeitet. Es werden stabile Dispersionen mit freien Nanosilber Partikeln erhalten.

In der Tabelle 4 sind die Ergebnisse mikrobiologischer Tests an Lacken auf Wasser- und Lösemittelbasis mit je 270 ppm Nanosilber angegeben. Bei beiden Lacken bewirkt eine Additivierung von 270 mg/kg Nanosilber eine starke Keimreduktion im Vergleich zur Standardoberfläche.

**Tabelle 4: Mikrobiologische Tests nach JIS 2801 an Lacken auf Wasser- und Lösemittelbasis mit je 270 ppm Nanosilber (zur Treppen- und Parkettversiegelung).**

| 1. Test Ergebnis | | | |
|---|---|---|---|
| Nach 0 h | Bakterienzahl Durchschnittswert [cfu] | | |
| Inoculum | 2,1x10⁵ | | |

| Nach 18 h | Bakterienzahl Durchschnittswert [cfu] | | F-Wert |
|---|---|---|---|
| interner Standard (Polystyrol) | 2,2x10⁵ | | 0,02 |

| 2-fach Bestimmung unabhängiger Proben | | | |
|---|---|---|---|
| Nach 18 h | Bakterienzahl Durchschnittswert [cfu] | % Reduktion ¹ | R-Wert |
| Lack, wasserbasiert mit 270 ppm Ag | 1,0x10² | 99,95% | 3,35 |
| Lack, lösemittelbasiert mit 270 ppm Ag | 1,0x10² | 99,95% | 3,35 |

| 2. Test Methode: | JIS Japanese Industrial Standard JIS Z 2801:2000 | | |
|---|---|---|---|
| | Antimicrobial products - Test for antimicrobial activity and efficacy. | | |
| | - Plate Count Method - | | |
| | | | |
| Test Stamm: | *Escherichia Coli* K12 | | |
| | | | |
| Modifikation: | Probengröße:25 mm x 25 mm | | |
| | Berechnung: nur R-Wert | | |
| | Preinkubation C: LB Broth | | |
| | Preinkubation D: LB Broth | | |
| | Inoculations-Medium:1/500 verd. LB Broth (+ 0,13 % Tween 80) | | |
| | Inkubation: 37°C | | |
| | Proben Präparation: UVC sterilisiert | | |

| | | | |
|---|---|---|---|
| 1) % Reduktion und R-Wert bezogen auf Internen Standard | | | |

### Beispiel 14:

### Verwendung der erfindungsgemäßen Formulierung in Silikonen

Die Nanosilber Dispersion aus Bsp. 1 wird in typischen Konzentrationen von 100 mg/kg bis 1000 mg/kg (bezogen auf den Silbergehalt im fertigen Produkt) wie aus dem Stand der Technik bekannt in handelsübliche Silikone eingearbeitet. Es werden stabile Dispersionen mit freien Nanosilber Partikeln erhalten.

In der Tabelle 5 sind die Ergebnisse mikrobiologischer Tests an 2-Komponenten Silikonen mit verschiedenen Gehalten an Nanosilber nach JIS 2802 angegeben. Bereits eine Additivierung von 200 mg/kg Nanosilber bewirkt eine starke Keimreduktion von 97,5 % im Vergleich zur Standardoberfläche. Ab 500 mg/kg Nanosilber wird eine starke Keimreduktion von 99,9 % erreicht.

**Tabelle 5: Mikrobiologische Tests nach JIS 2801 an 2-Komponenten Silikonen mit verschiedenen Gehalten an Nanosilber**

| 1. Test Ergebnis | | | |
|---|---|---|---|
| Nach 0 h | Bakterienzahl Durchschnittswert [cfu] | | |
| Inoculum | 1,0x10⁵ | | |

| Nach 18 h | Bakterienzahl Durchschnittswert [cfu] | | F-Wert |
|---|---|---|---|
| interner Standard (Polystyrol) | 1,4x10⁵ | | 0,02 |

| 2-fach Bestimmung unabhängiger Proben | | | |
|---|---|---|---|
| Nach 18 h | Bakterienzahl Durchschnittswert [cfu] | % Reduktion ¹ | R-Wert |
| Silikon mit 200 ppm Ag | 3,5x10³ | 97,5% | 1,6 |
| Silikon mit 500 ppm Ag | <1,0x10² | 99,9 | 3,1 |
| Silikon mit 1000 ppm Ag | <1,0x10² | 99,9% | 3,1 |

| 2. Test Methode: | JIS Japanese Industrial Standard JIS Z 2801:2000 | | |
|---|---|---|---|
| | Antimicrobial products - Test for antimicrobial activity and efficacy. | | |
| | - Plate Count Method - | | |
| | | | |
| Test Stamm: | *Escherichia Coli* K12 | | |
| | | | |
| Modifikation: | Probengröße:25 mm x 25 mm | | |
| | Berechnung: nur R-Wert | | |
| | Preinkubation C: LB Broth | | |
| | Preinkubation D: LB Broth | | |
| | Inoculations-Medium:1/500 verd. LB Broth (+0,13 % Tween 80) | | |
| | Inkubation: 37°C | | |
| | Proben Präparation: UVC sterilisiert | | |

| | | | |
|---|---|---|---|
| 1)% Reduktion und R-Wert bezogen auf Internen Standard | | | |

### Beispiel 15:

### Verwendung der erfindungsgemäßen Formulierung in Polypropylen Folien

Die Nanosilber Dispersion aus Beispiel 1 wird in typischen Konzentrationen von 100 mg/kg bis 5000 mg/kg (bezogen auf den Silbergehalt in der fertigen Schicht) in eine Schicht einer mehrschichtigen Polypropylenfolie mittels Extrusion eingearbeitet. Es werden Folien mit in einer etwa 5 pm starken Schicht homogen verteilten, überwiegend isolierten Nanosilber Partikeln erhalten.

In der Tabelle 6 sind die Ergebnisse mikrobiologischer Tests an einer mehrschichtigen Polypropylenfolie mit verschiedenen Gehalten an Nanosilber nach JIS 2801 angegeben. Die Additivierung mit 2100 mg/kg bzw. 3200 mg/kg Nanosilber bewirkt jeweils eine starke Keimreduktion von etwa 99 % im Vergleich zur Standardoberfläche.

**Tabelle 6: Mikrobiologische Tests an einer mehrschichtigen Polypropylenfolie.**

| 1. Test Ergebnis | | | |
|---|---|---|---|
| Nach 0 h | Bakterienzahl Durchschnittswert [cfu] | | |
| Inoculum | 1,7x10⁵ | | |

| Nach 18 h | Bakterienzahl Durchschnittswert [cfu] | | F-Wert |
|---|---|---|---|
| interner Standard | (Polystyrol) 5,1x10⁵ | | 0,5 |

| 2-fach Bestimmung unabhängiger Proben | | | |
|---|---|---|---|
| Nach 18 h | Bakterienzahl Durchschnittswert [cfu] | % Reduktion ¹ | R-Wert |
| Kontrolle, antibakteriell | <1,0x10² | >99,98 % | > 3,7 |
| Folie mit 2100 ppm Ag auf der Innenseite | 5,8x10³ | 98,87 % | 1,9 |
| Folie mit 3200 ppm Ag auf der Innenseite | 6,9x10³ | 98,66 % | 1,9 |
| 1)% Reduktion und R-Wert bezogen auf internen Standard | | | |

| 2. Test Methode: | JIS Japanese Industrial Standard JIS Z 2801:2000 | | |
|---|---|---|---|
| | Antimicrobial products - Test for antimicrobial activity and efficacy. | | |
| | - Plate Count Method - | | |
| | | | |
| Test Stamm: | *Escherichia Coli K12* | | |
| Modifikation: | Probengröße:30 mm x 40 mm | | |
| | Berechnung: nur R-Wert | | |
| | Preinkubation: LB-Medium | | |
| | Inoculations-Medium:1/500 verd. LB Broth (+0,13 % Tween 80) | | |
| | Inkubation: 37°C | | |
| | Proben Präparation: nein | | |

### Beispiel 16:

### Verwendung der erfindungsgemäßen Formulierung in Polypropylen Flottenbehälter

Die Nanosilber Dispersion aus Beispiel 1 wird in einer Konzentration von 6500 mg/ (bezogen auf den Silbergehalt) mittels Extrusion in Polypropylen eingearbeitet. Dabei wird ein Masterbatch mit überwiegend isoliert voneinander vorliegenden Nanosilberpartikeln erhalten. Das Nanosilber Masterbatch wird in typischen Konzentrationen von 100 mg/kg bis 5000 mg/kg (bezogen auf den Silbergehalt im fertigen Polymer) in Polypropylen Flottenbehälter eingearbeitet. Die Flottenbehälter sind zur Aufnahme und Zwischenspeicherung gebrauchter Waschlauge bzw. Spüllösungen aus Geschirr- bzw. Textilwaschprozessen bestimmt. Die Additivierung von Nanosilber soll die Besiedelung des Polymers durch Keime verhindern.

In der Tabelle 7 sind die Ergebnisse mikrobiologischer Tests an Polypropylen-Flottenbehältern nach JIS 2801 mit verschiedenen Gehalten an Nanosilber angegeben. Die Additivierung mit 520 mg/kg bzw. 1000 mg/kg bzw. 2000 mg/kg Nanosilber bewirkt jeweils eine starke Keimreduktion von mehr als 99,99 % im Vergleich zur Standardoberfläche.

**Tabelle 7: Mikrobiologische Tests an Polypropylen-Flottenbehältern nach JIS 2801**

| 1. Test Ergebnis | | | |
|---|---|---|---|
| Nach 0 h | Bakterienzahl Durchschnittswert [cfu] | | |
| Inoculum | 3,6x10⁵ | | |

| Nach 18 h | Bakterienzahl Durchschnittswert [cfu] | | F-Wert |
|---|---|---|---|
| interner Standard (Polystyrol) | 1,1x10⁶ | | 0,5 |

| 2 fold parallel independent determinations | | | |
|---|---|---|---|
| Nach 18 h | Bakterienzahl Durchschnittswert [cfu] | % Reduktion ¹ | R-Wert |
| Kontrolle, antibakteriell | <1,0x10² | >99,99 % | > 4,0 |

| 3-fach Bestimmung unabhängiger Proben | | | |
|---|---|---|---|
| Flottenbehälter mit 520 ppm Ag | <1,0x10² | >99,99 % | > 4,0 |
| Flottenbehälter mit 1000 ppm Ag | <1,0x10² | >99,99 % | > 4,0 |
| Flottenbehälter mit 2000 ppm Ag | <1,0x10² | >99,99 % | > 4,0 |
| | | | |

| 2. Test Methode: | JIS Japanese Industrial Standard JIS Z 2801:2000 | | |
|---|---|---|---|
| | Antimicrobial products - Test for antimicrobial activity and efficacy. | | |
| | - Plate Count Method - | | |
| | | | |
| Test Stamm: | *Escherichia Coli K12* | | |
| Modifikation: | Probengröße: 30 mm x 40 mm | | |
| | Berechnung: nur R-Wert | | |
| | Preinkubation: LB-Medium | | |
| | Inoculations-Medium:1/500 verd. LB Broth (+0,13 % Tween 80) | | |
| | Inkubation: 37°C | | |
| | Proben Präparation: nein | | |

| | | | |
|---|---|---|---|
| 1)% Reduktion und R-Wert bezogen auf internen Standard | | | |

### Beispiel 17:

### Verwendung der erfindungsgemäßen Formulierung in Wood Plastic Composites (WPC)

Die Nanosilber Dispersion aus Beispiel 1 wird bei der Extrusion von PVC und Holzmehl in einer Konzentration von 50 mg/kg bis 1000 mg/kg zugemischt. Es werden witterungs- und fäulnisbeständige WPC Materialien erhalten.

### Beispiel 18:

### Verwendung der erfindungsgemäßen Formulierung in Polyolefin Formkörper

Die Nanosilber Dispersion aus Beispiel 1 wird unter Zuhilfenahme von Weißöl mit Polymerkügelchen aus Polyolefinen (PE bzw. PP) gemischt, so dass eine Silberkonzentration von 50 mg/kg bis 500 mg/kg in der fertigen Mischung erreicht wird. Die Mischung wird in einer Presse in Formkörper (z.B. Schneidbretter, Filter, Kosmetikapplikatoren) gepresst und mechanisch nachbearbeitet. Die resultirenden Schneidbretter besitzen eine antimikrobielle Aktivität mit einem R-Wert zwischen 1 und 4, je nach Silbergehalt.

### Beispiel 19:

### Verwendung der erfindungsgemäßen Formulierung zur Herstellung von PVC Plastisol beschichteten Textilien

Die Nanosilber Dispersion aus Beispiel 1 wird in typischen Konzentrationen von 400 mg/kg (bezogen auf den Silbergehalt im fertigen Polymer) in PVC Plastisol eingearbeitet. Das Plastisol wird zur Beschichtung textiler Gewirke verwendet. Es entstehen Matten, die sich beispielsweise als Bodenbelag in Feuchträumen, als Gymnastikmatte, als Teppichstopp oder auch als Geschirrablage einsetzen lassen. Die Additivierung von Nanosilber soll die Besiedelung des Polymers durch Mikroorganismen verhindern.
In der Tabelle 8 sind die Ergebnisse mikrobiologischer Tests an Polypropylen-Flottenbehältern mit einer Additivierung von 400 mg/kg Nanosilber angegeben. Die Additivierung mit 400 mg/kg Nanosilber bewirkt eine signifikante antimycotische Aktivität gegen die 5 getesteten Pilze.

**Tabelle 8: Test auf antimycotische Aktivität von PVC Plastisol mit einem Silbergehalt von 400ppm.**

| 1. Test Ergebnis | | |
|---|---|---|
| Kontrolle | Pilz Wachstum | Kat. |
| Baumwolle (interner | Standard) stark | 6 |
| Wachstums-Kontrolle | stark | 6 |
| Kontrolle, Sterilität | nein | |

| Probe | Antimycot. Aktivität | Kat. |
|---|---|---|
| PVC Plastisol gewaschen | signifikant | 5 |
| PVC Plastisol unbehandelt, 15x mit 400 ppm Ag, 15x gewaschen | nein | 3 |
| | | |

| 2. Test Methode | SN 195921 - Textile Fabric - Determination of the Antimycotic Activity - modified | |
|---|---|---|
| Test Stämme: | DSM 40464 - *Streptomyces abikoensis* | |
| | DSM 9122 - *Scopulariopsis brevicaulis* | |
| | | |
| | DSM 62413 - *Fusarium solani* | |
| | DSM 10640 - *Penicillium funiculosum* | |
| | DSM 2404 - *Aureobasidium pullulans* | |
| | | |
| Modifikation: | Probengröße: 30 mm Ø | |
| | Preinkubation: Potato Dextrose Agar | |
| | Suspensions-Medium: Potato Dextrose Bouillon | |
| | Proben-Inoculation: Sprühen | |
| | Inkubation: 25°C | |
| | Inkubations-Zeit: 41 Tage | |
| | Proben-Sterilisation: UVC | |

### Beispiel 20:

### Verwendung der erfindungsgemäßen Formulierung in PMMA Knochenzement

Die Nanosilber Dispersion aus Beispiel 1 wird in typischen Konzentrationen von 100 mg/kg bis 5000 mg/kg (bezogen auf den Silbergehalt im fertigen Produkt) mit den Mitteln des Standes der Technik in handelsübliche PMMA Knochenzemente eingearbeitet. Die Einarbeitung kann entweder in das trockene PMMA Pulver oder in das flüssige MMA Monomer erfolgen. Nach der Aushärtung werden Knochenzemente mit einer homogenen Verteilung überwiegend isolierter Nanosilber Partikel erhalten.

In der Tabelle 9 sind die Ergebnisse verschiedener Elutionstests angegeben.
So ist der Tabelle 9 zu entnehmen, dass ein mit 2149 mg/kg ausgerüsteter Knochenzement Prüfkörper bei einer Elution mit 10 ml SimulatedBodyFluid (SBF) bei einer Elutionstemperatur von 37 °C innerhalb von 12 Tagen 4 ng Silber pro mm² Fläche in die Lösung verliert. Aus der folgenden Zeile der Tabelle 9 ist zu entnehmen, dass eine leichte Erhöhung des Nanosilber Gehaltes im Prüfkörper auf 2500 mg/kg und eine Verkürzung der Elutionszeit auf 5 Tage keine nennenswerte Veränderung der eluierten Silbermenge bewirkt. Bei einem Alterungsversuch wurde mittels Kochen des Prüfkörpers in SBF eine Menge von 13 ng/mm² eluiert. Bei tgl. Erneuerung der Elutionsflüssigkeit stellt sich ein Gleichgewicht von 1,2 ng/mm² und Tag ein.

**Tabelle 9: Acrylat-Knochenzement (PMMA) mit 2500 ppm Ag.**

| | nAg Gehalt im Prüfkörper | Elutionsvolumen | Elutionstemp. | Elutionsdauer | Eluierte Silbermenge |
|---|---|---|---|---|---|
| Freisetzungskinetik | 2149 ppm | 10 mL SBFm | 37 °C | 12 d | 4 ng/mm² (0,4 ng/g mm²) |
| Variation d.Silbergehalte | 2500 ppm | 10 mL SBFm | 37 °C | 5 d | 4 ng/mm² (0,4 ng/g*mm²) |

### Beispiel 21:

### Verwendung der erfindungsgemäßen Formulierung zur Herstellung von PMMA Beschichtungen

Die Nanosilber Dispersion aus Beispiel 1 wird in typischen Konzentrationen von 100 mg/kg bis 5000 mg/kg (bezogen auf den Silbergehalt im fertigen Produkt) wie aus dem Stand der Technik bekannt in handelsübliche PMMA Zubereitungen eingearbeitet. Die Einarbeitung kann entweder in das trockene PMMA Pulver oder in das flüssige MMA Monomer erfolgen. Die angemischten Zubereitungen werden zur Beschichtung vorwiegend medizinischer Produkte eingesetzt. Nach der Aushärtung werden PMMA Beschichtungen mit einer homogenen Verteilung überwiegend isolierter Nanosilber Partikel erhalten.

### Beispiel 22:

### Verwendung der erfindungsgemäßen Formulierung zur Herstellung von Synthese Fasern

Die Nanosilber Dispersion aus Beispiel 1 wird in typischen Konzentrationen von 1000 mg/kg bis 20000 mg/kg (bezogen auf den Silbergehalt) mittels Extrusion in handelsübliche Thermoplaste wie z.B. Polypropylen, Polyester, Polyamid eingearbeitet. Dabei werden Masterbatches mit überwiegend isoliert voneinander vorliegenden Nanosilberpartikeln erhalten.

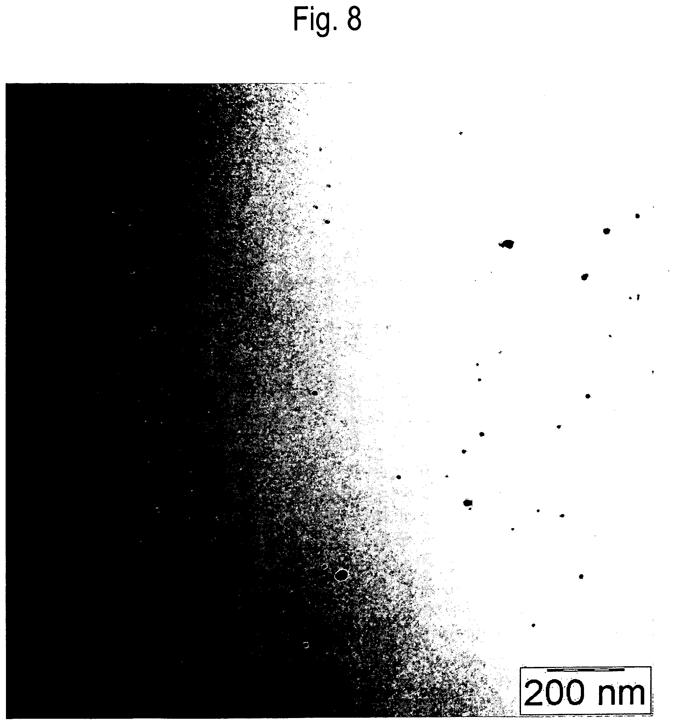

Figur 8 zeigt beispielsweise die TEM-Aufnahme eines Polyester Masterbatches mit 6500 mg/kg Silber. Die dunklen Punkte in Figur 8 zeigen die homogene Verteilung und die geringe Agglomeration der Nanosilberpartikel im Polyester.

Die Masterbatches werden dem Stand der Technik entsprechend verdünnt zur Herstellung von Synthesefasern z.B. aus Polypropylen, Polyester oder Polyamid eingesetzt.

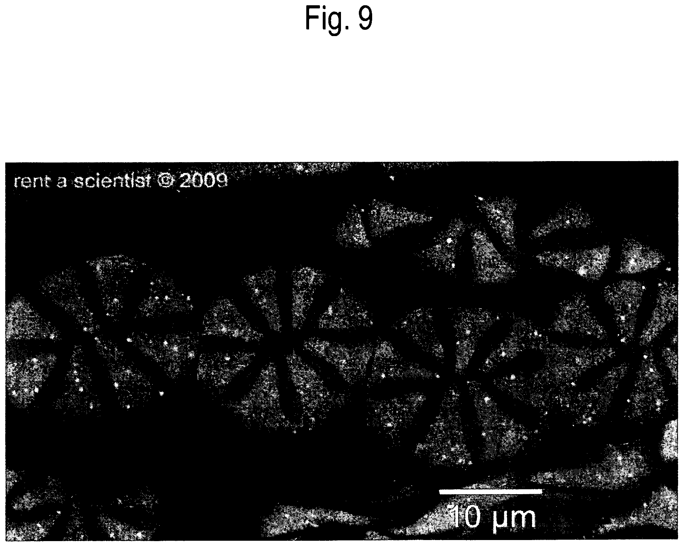

Figur 9 zeigt mehrere Microfaserstränge aus PET/PA mit 200 mg/kg Nanosilber. In der Figur 9 sind die einzelnen Nanosilberpartikel (helle Punkte) in den segmentierten Garnen deutlich zu erkennen. Typische Silbergehalte für antimikrobielle Effekte liegen im Bereich von 100 mg/kg bis 300 mg/kg.

Neben Mikrofasern werden auch Monofil und Bikomponentenfasern für Bekleidung, Bettenfüllungen, Tücher und technische Textilien sowie nonwoven Materialien hergestellt. Die silberhaltigen Synthesefasern können auch in Form von Stapelfasern zur Ausrüstung anderer Fasern (auch Naturfasern wie z.B. Baumwolle) eingesetzt werden. Die eingesetzten Silbergehalte liegen entsprechend der Verdünnung durch andere Fasern auf einem höheren Niveau im Vergleich zur direkten Ausrüstung der Synthesefasern.

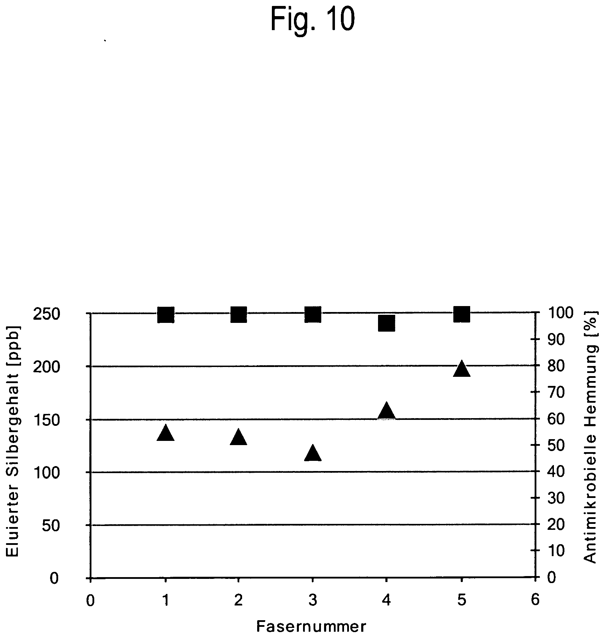

Figur 10 zeigt das Elutionsverhalten (**▲**) und die antimikrobielle Aktivität (■) verschiedener Polyester Mikrofasern. Der Silbergehalt der verschiedenen Fasern liegt im Bereich von 150 mg/kg bis195 mg/kg. Die Fasern wurden jeweils für 3 h in Wasser eluiert. Der eluierte Silbergehalt liegt im Bereich von 120 pg/kg bis 200 pg/kg Wasser; die antimikrobielle Hemmung liegt jeweils über 96 %. In Tabelle 10 sind die Ergebnisse der antimikrobiellen Tests nach JIS 1902 dieser 5 Mikrofasern dargestellt.

### Beispiel 23:

### Herstellung einer stabilen Dispersion von Silbernanopartikel in Methylmethacrylat

Für die Herstellung einer Formulierung von Silbernanopartikel in Methylmethacrylat wird das Produkt aus Beispiel 6 oder Beispiel 7 verwendet. Dazu werden 990 g Methylmethacrylat (Merck, zur Synthese) in einem 2 L Becherglas vorgelegt und auf dem Magnetrührer bei Raumtemperatur gerührt. Mit einer Pipette werden 1 g Netzmittel (Evonic, Tego dispers 655) zu gegeben. Nach Zugabe von 9,3 g des Produktes aus Beispiel 6 verändert sich die Farbe der Lösung zu Orangebraun. Durch Schwenken des Glases kann ein dünner Film an der Glaswand generiert werden, der hellgelb, klar gefärbt ist und keine sichtbaren Teilchen enthält. Es werden 1.000 g einer Dispersion erhalten mit einem Silbergehalt von 5.115 mg/kg. Partikelgröße und -verteilung entspricht der Darstellung in den Figuren 2 und 3.

### Beispiel 24:

### Herstellung einer stabilen Dispersion von Silbernanopartikel in Methylmethacrylat

Für die Herstellung einer Formulierung von Silbernanopartikel in Methylmethacrylat wird das Produkt aus Beispiel 6 oder Beispiel 7 verwendet. Dazu werden 980 g Methylmethacrylat (Merck, zur Synthese) in einem 2 L Becherglas vorgelegt und auf dem Magnetrührer bei Raumtemperatur gerührt. Mit einer Pipette werden 2 g Netzmittel (Evonic, Tego dispers 655) zu gegeben. Nach der Zugabe von 18,2 g des Produktes aus Beispiel 6 verändert sich die Farbe der Lösung zu Orangebraun. Durch Schwenken des Glases kann ein dünner Film an der Glaswand generiert werden, der hellgelb, klar gefärbt ist und keine sichtbaren Teilchen enthält. Es werden 1.000 g einer Dispersion erhalten mit einem Silbergehalt von 10.010 mg/kg. Partikelgröße und -verteilung entspricht der Darstellung in den Figuren 2 und 3.

### Beispiel 25:

### Herstellung einer stabilen Dispersion von Silbernanopartikel in Methylmethacrylat

Für die Herstellung einer Formulierung von Silbernanopartikel in Methylmethacrylat wird das Produkt aus Beispiel 6 oder Beispiel 7 verwendet. Dazu werden 897 g Methylmethacrylat (Merck, zur Synthese) in einem 2 L Becherglas vorgelegt und auf dem Magnetrührer bei Raumtemperatur gerührt. Mit einer Pipette werden 10 g Netzmittel (Evonic, Tego dispers 655) zu gegeben. Nach der Zugabe von 92,7 g des Produktes aus Beispiel 6 verändert sich die Farbe der Lösung zu Orangebraun. Durch Schwenken des Glases kann ein dünner Film an der Glaswand generiert werden, der hellgelb, klar gefärbt ist und keine sichtbaren Teilchen enthält. Es werden 1.000 g einer Dispersion erhalten mit einem Silbergehalt von 50.985 mg/kg. Partikelgröße und -verteilung entspricht der Darstellung in den Figuren 2 und 3.

### Beispiel 26:

### Herstellung eines antibakteriellen Trägermaterials auf PMMA-Basis

Das Produkt aus Bespiel 1 wird in einer Konzentration von 100 mg/kg bis 10.000 mg/kg, jeweils bezogen auf das fertige Produkt, in ein PMMA-Perlpolymerisat eingearbeitet. Vorzugsweise erfolgt die Einarbeitung in das trockene PMMA Pulver. Dem PMMA Pulver kann des Weiteren noch eine pharmazeutisch wirksame Substanz wie Gentamicin sowie weiteren Zusatzstoffe wie beispielsweise Zirkoniumoxid als Röntgenkontrastmittel zugesetzt werden.

Aus dem PMMA-Pulver werden im Spritzgussverfahren antimikrobiell wirksame Kugeln beispielsweise mit einem Durchmesser zwischen 5 und 10 mm und einem Gewicht von 100 bis 300 mg hergestellt. Die ist aufgrund der Hitzebeständigkeit der Nanosilberdispersion bis 240 °C ohne weiteres möglich. Eine Kugel von 200 mg kann beispielsweise 4,5 g Gentamicin und 20 mg Zirkoniumoxid enthalten.

Die Kugeln können auf einem multifilen chirurgische Draht verankert werden.

## Patentansprüche

1. Verfahren zur Herstellung einer silbernanopartikelhaltigen Dispersion, insbesondere zur Herstellung von Knochenzement oder eines Beschichtungsmittels für Implantate oder medizinische Instrumente oder eines antibakteriellen Trägermaterials umfassend die Schritte
- Bereitstellen eines Silbersalzes,
- Bereitstellen zumindest eines Stabilisators,
- Bereitstellen eines Reduktionsmittels,
- Bereitstellen eines organischen polymerisierbaren Lösungsmittels,
- Herstellen einer Lösung von Silbersalz, Stabilisator und Reduktionsmittel, welche Wasser enthält,
- Zugabe einer Base zu der Lösung,
- Zugabe eines anorganischen Salzes,
wobei mit Zugabe der Base Silbernanopartikel ausfallen, welche dispergieren,
wobei über das anorganische Salz das in der Lösung verbleibende Wasser hydratisiert wird und sich eine wässrige Phase bildet, wohingegen die Silbernanopartikel aufgrund des zugesetzten Stabilisators überwiegend in dem organischen polymerisierbaren Lösungsmittel verbleiben,
- Abtrennen der sich nach Zugabe des anorganischen Salzes bildenden wässrigen Phase von der Silbernanopartikel und Stabilisatoren umfassenden Mischung, so dass ein organisches Lösungsmittel mit Silbernanopartikeln zurückbleibt.

2. Verfahren zur Herstellung einer silbernanopartikelhaltigen Dispersion nach dem vorstehenden Anspruch, **dadurch gekennzeichnet, dass** zu der Silbernanopartikel und Stabilisatoren umfassenden Mischung zumindest ein Netz- und Dispergieradditiv hinzugegeben wird.

3. Verfahren zur Herstellung einer silbernanopartikelhaltigen Dispersion nach einem der vorstehenden Ansprüche, **dadurch gekennzeichnet, dass** der zumindest eine Stabilisator ausgewählt aus der Gruppe bestehend aus Polyoxyethylen-mono-alkylsäureester, Polyoxypropylen-mono-alkylsäureester, Polyoxyethylen-di-alkylsäureester, Polyoxypropylen-di-alkylsäureester, Polyoxyethylen-tri-alkylsäureester, Polyoxypropylen-tri-alkylsäureester und deren Gemische.

4. Verfahren zur Herstellung einer silbernanopartikelhaltigen Dispersion nach einem der vorstehenden Ansprüche, **dadurch gekennzeichnet, dass** als organisches polymerisierbares Lösungsmittel ein Acrylat, insbesondere Methylmethacrylat verwendet wird.

5. Verfahren zur Herstellung einer silbernanopartikelhaltigen Dispersion nach einem der vorstehenden Ansprüche, **dadurch gekennzeichnet, dass** die Zugabe der Base kontinuierlich, insbesondere über einen Zeitraum von 5 bis 48 h, derart erfolgt, dass der pH- Wert der Formulierung zwischen 0 und 6 liegt.

6. Verfahren zur Herstellung einer silbernanopartikelhaltigen Dispersion nach einem der vorstehenden Ansprüche, **dadurch gekennzeichnet, dass** die wässrige Phase von der Silbernanopartikel und Stabilisatoren umfassenden silbernanopartikelhaltigen Mischung durch Abdekantieren getrennt wird.

7. Verfahren nach einem der vorstehenden Ansprüche, wobei das Reduktionsmittel mit den Silberionen des Silbersalzes unter Bildung von elementarem Silber und ansonsten überwiegend gasförmigen Reaktionsprodukten reagiert und/oder wobei als Reduktionsmittel Hydrazinhydrat eingesetzt wird und/oder wobei als Base eine Base mit einem pKb Wert im Bereich von -2 bis 10,5, bevorzugt mit einem pKb Wert im Bereich von 1,5 bis 9,1, besonders bevorzugt mit einem pKb Wert im Bereich 3,5 bis 7,5 eingesetzt wird und/oder wobei als Base Ammoniak, Kaliumhydrogencarbonat oder Natriumhydroxid eingesetzt wird und/oder wobei die Zugabe der Base kontinuierlich über einen Zeitraum von 9 bis 30 h erfolgt und/oder wobei das anorganische Salz zumindest ein Kation der Periode 3 oder der Periode 4 des Periodensystems der Elemente und zumindest ein Element der fünften Hauptgruppe des Periodensystems der Elemente als Bestandteil des Anions umfasst und/oder wobei das anorganische Salz Stickstoff als Bestandteil des Anions umfasst und/oder wobei es sich bei dem Netz- und Dispergieradditiv um ein Alkylphenolethoxylat, einen aminofunktionellen Polyester, eine phosphorhaltige Substanz oder eine Mischung dieser Verbindungen handelt und/oder wobei es sich bei dem Netz- und Dispergieradditiv um ein organisch modifiziertes Phosphat, ein Phosphonat, eine Polyphosphorverbindung, ein Alkylphosphonat, eine Phosphorverbindung mit gemischten organischen Liganden, ein Oligomer oder ein Polymer mit phosphathaltigen Liganden handelt.

8. Silbernanopartikelhaltige Dispersion herstellbar mit einem Verfahren nach einem der vorstehenden Ansprüche, umfassend Silbernanopartikel,
zumindest einen Stabilisator ausgewählt aus der Gruppe bestehend aus Polyoxyethylen-mono-alkylsäureester, Polyoxypropylen-mono-alkylsäureester, Polyoxyethylen-di-alkylsäureester, Polyoxypropylen-di-alkylsäureester, Polyoxyethylen-tri-alkylsäureester, Polyoxypropylen-tri-alkylsäureester und deren Gemische,
und zumindest ein nicht ionisches Tensid als ein Netz- und Dispergieradditiv, wobei die Silbernanopartikel in einem flüssigen Monomer, Prepolymer oder Polymer dispergiert sind,
und wobei die Silbernanopartikel von dem zumindest einen Stabilisator und von dem Netz- und Dispergieradditiv umhüllt sind.

9. Silbernanopartikelhaltige Dispersion nach Anspruch 8, **dadurch gekennzeichnet, dass** es sich bei dem Netz- und Dispergieradditiv um ein organisch modifiziertes Phosphat, ein Phosphonat, eine Polyphosphorverbindung, ein Alkylphosphonat, eine Phosphorverbindung mit gemischten organischen Liganden, ein Oligomer oder ein Polymer mit phosphathaltigen Liganden handelt.

10. Silbernanopartikelhaltige Dispersion nach einem der vorstehenden Ansprüche 8 oder 9, **dadurch gekennzeichnet, dass** die mittlere Partikelgröße der Nanopartikel zwischen 5 und 50, vorzugsweise zwischen 10 und 20 nm liegt und/oder dass zumindest 90, vorzugsweise zumindest 99 % der Silbernanopartikel kleiner als 50, vorzugsweise kleiner als 20 nm sind, und/oder dass die Silbernanopartikel eine im Wesentlichen sphärische Form aufweisen und/oder dass der Anteil an Silbernanopartikeln zwischen 0,5 und 5, vorzugsweise zwischen 1 und 3 Gewichts% beträgt und/oder dass das Polymer ein Acrylat oder eine Acyrylatvorstufe, insbesondere Methylmethacrylat umfasst.

11. Verwendung einer silbernanopartikelhaltigen Dispersion nach einem der vorstehenden Ansprüche 8 bis 10 als Monomer, insbesondere für Knochenzement, Beschichtungslösung oder Zuschlagstoff für Polymerwerkstoffe, insbesondere für Kunststoffimplantate.

12. Verwendung einer silbernanopartikelhaltigen Mischung umfassend Silbernanopartikel nach Anspruch 11, wobei in der silbernanopartikelhaltigen Mischung zumindest zwei Stabilisatoren anwesend sind, ausgewählt aus der Gruppe bestehend aus Polyoxyethylen-mono-alkylsäureester, Polyoxypropylen-mono-alkylsäureester, Polyoxyethylen-di-alkylsäureester, Polyoxypropylen-di-alkylsäureester, Polyoxyethylen-tri-alkylsäureester und Polyoxypropylen-tri-alkylsäureester, und wobei das Mengenverhältnis Silbernanopartikel zu Stabilisator im Bereich von 10 : 2 bis zu 10 : 50 liegt zur Herstellung von Knochenzement oder eines Beschichtungsmittels für Implantate und/oder medizinische Instrumente oder eines antibakteriellen Trägermaterials.

13. Verwendung nach dem vorstehenden Anspruch 12, wobei die Stabilisatoren ausgewählt sind aus der Gruppe bestehend aus Polyoxyethylen-Sorbitan-Monolaurat, Polyoxyethylen-Sorbitan-Monopalmitat, Polyoxyethylen-Sorbitan-Monostearat, Polyoxyethylen-Sorbitan-Monooleat, Polyoxyethylen-SorbitanTristearat, Polyoxyethylen-Glyceryl-Trioleat, Polyoxyethylen-Glyceryl-Monolaurat, Polyoxyethylen-Glyceryl-Monooleat, Polyoxyethylen-Glyceryl-Monostearat, Polyoxyethylen-Glyceryl-Monoricinoleat, Rizinusöl, hydriertes Rizinusöl, Sojabohnenöl und deren Gemische und/oder wobei die Stabilisatoren in einem Mengenverhältnis im Bereich von 1 : 1 bis 2 : 1 anwesend sind und/oder wobei die Silbernanopartikel in einer Partikelgröße von 1 bis 100 nm, bevorzugt 1 bis 50 nm, besonders bevorzugt 1 bis 20 nm vorliegen.

## Claims

1. A method for producing a dispersion containing silver nanoparticles, in particular for producing bone cement or a coating agent for implants or medical instruments, or an antibacterial carrier material, comprising the steps of:
- providing a silver salt;
- providing at least one stabiliser;
- providing a reducing agent;
- providing an organic polymerisable solvent;
- producing an aqueous solution of silver salt, stabiliser, and reducing agent;
- adding a base to the solution;
- adding an inorganic salt;
wherein when adding said base, silver nanoparticles are precipitated and dispersed;
wherein the water remaining in the solution is hydrated by said inorganic salt so that an aqueous phase is formed, whereas the silver nanoparticles predominantly remain in the organic polymerisable solvent, due to the added stabiliser;
- separating the aqueous phase that is formed after adding the inorganic salt from the mixture including silver nanoparticles and stabilisers, such that an organic solvent including silver nanoparticles is remaining.

2. The method for producing a dispersion containing silver nanoparticles according to the preceding claim, **characterised in that** at least one wetting and dispersing additive is added to said mixture including silver nanoparticles and stabilisers.

3. The method for producing a dispersion containing silver nanoparticles according to any one of the preceding claims, **characterised in that** the at least one stabiliser is selected from the group consisting of polyoxyethylene mono-alkyl acid ester, polyoxypropylene mono-alkyl acid ester, polyoxyethylene di-alkyl acid ester, polyoxypropylene di-alkyl acid ester, polyoxyethylene tri-alkyl acid ester, polyoxypropylene tri-alkyl acid ester, and mixtures thereof.

4. The method for producing a dispersion containing silver nanoparticles according to any one of the preceding claims, **characterised in that** an acrylate is used as the organic polymerisable solvent, in particular methyl methacrylate.

5. The method for producing a dispersion containing silver nanoparticles according to any one of the preceding claims, **characterised in that** said base is added continuously, in particular over a time interval of 5 to 48 hours, such that the pH value of the formulation is between 0 and 6.

6. The method for producing a dispersion containing silver nanoparticles according to any one of the preceding claims, **characterised in that** the aqueous phase is separated from the mixture including silver nanoparticles and stabilisers by decanting.

7. The method according to any one of the preceding claims, wherein the reducing agent reacts with the silver ions of the silver salt to form elemental silver and otherwise predominantly gaseous reaction products; and/or wherein hydrazine hydrate is used as a reducing agent; and/or wherein a base having a pK_{b} value in a range from -2 to 10.5 is used as the base, preferably with a pK_{b} value in a range from 1.5 to 9.1, most preferably with a pK_{b} value in a range from 3.5 to 7.5; and/or wherein ammonia, potassium hydrogencarbonate or sodium hydroxide is used as the base; wherein the base is added continuously over a time interval of 9 to 30 hours; and/or wherein the inorganic salt comprises at least one cation of period 3 or period 4 of the periodic table of the elements and at least one element of IUPAC group 15 of the periodic table of the elements as a component of the anion; and/or wherein the inorganic salt comprises nitrogen as a component of the anion; and/or wherein the wetting and dispersing additive is an alkylphenol ethoxylate, an amino-functional polyester, a phosphorus-containing substance or a mixture of these compounds; and/or wherein the wetting and dispersing additive is an organically modified phosphate, a phosphonate, a polyphosphorus compound, an alkylphosphonate, a phosphorus compound comprising mixed organic ligands, an oligomer or a polymer comprising phosphate-containing ligands.

8. A dispersion containing silver nanoparticles, producible by a method according to any one of the preceding claims, comprising:
silver nanoparticles;
at least one stabiliser, selected from the group consisting of polyoxyethylene mono-alkyl acid ester, polyoxypropylene mono-alkyl acid ester, polyoxyethylene di-alkyl acid ester, polyoxypropylene di-alkyl acid ester, polyoxyethylene tri-alkyl acid ester and polyoxypropylene tri-alkyl acid ester and mixtures thereof; and
at least one non-ionic surfactant as a wetting and dispersing additive, wherein the silver nanoparticles are dispersed in a liquid monomer, pre-polymer or polymer; and
wherein the silver nanoparticles are enveloped by the at least one stabiliser and by the wetting and dispersing additive.

9. The dispersion containing silver nanoparticles according to claim 8, **characterised in that** the wetting and dispersing additive is an organically-modified phosphate, a phosphonate, a polyphosphorus compound, an alkylphosphonate, a phosphorus compound comprising mixed organic ligands, an oligomer or a polymer comprising phosphate-containing ligands.

10. The dispersion containing silver nanoparticles according to claim 8 or 9, **characterised in that** the nanoparticles have a mean particle size between 5 and 50 nm, preferably between 10 and 20 nm; and/or that at least 90 %, preferably 99 % of the silver nanoparticles are smaller than 50 nm, preferably smaller than 20 nm; and/or that the silver nanoparticles have a substantially spherical shape; and/or that the fraction of silver nanoparticles is between 0.5 and 5 wt%, preferably between 1 and 3 wt%; and/or that the polymer comprises an acrylate or an acrylate precursor, in particular methyl methacrylate.

11. Use of a dispersion containing silver nanoparticles according to any one of the preceding claims 8 to 10 as a monomer, in particular for bone cement, a coating solution or an additive for polymer materials, in particular for polymer implants.

12. Use of a silver nanoparticles-containing mixture comprising silver nanoparticles according to claim 11, wherein the nanoparticles-containing mixture includes at least two stabilizers which are selected from the group consisting of polyoxyethylene mono-alkyl acid ester, polyoxypropylene mono-alkyl acid ester, polyoxyethylene di-alkyl acid ester, polyoxypropylene di-alkyl acid ester, polyoxyethylene tri-alkyl acid ester and polyoxypropylene tri-alkyl acid ester, and wherein a quantitative ratio of silver nanoparticles to stabilizer is in a range from 10:2 to 10:50, for producing bone cement or a coating agent for implants and/or medical instruments or an antibacterial carrier material.

13. Use according to the preceding claim 12, wherein the stabilisers are selected from the group consisting of polyoxyethylene sorbitan monolaurate, polyoxyethylene sorbitan monopalmitate, polyoxyethylene sorbitan monostearate, polyoxyethylene sorbitan monooleate, polyoxyethylene sorbitan tristearate, polyoxyethylene glyceryl trioleate, polyoxyethylene glyceryl monolaurate, polyoxyethylene glyceryl monooleate, polyoxyethylene glyceryl monostearate, polyoxyethylene glyceryl monoricinoleate, castor oil, hydrogenated castor oil, soybean oil and mixtures thereof; and/or wherein the stabilisers are present in a quantitative ratio in a range from 1:1 to 2:1; and/or wherein the silver nanoparticles are present in a particle size from 1 to 100 nm, preferably 1 to 50 nm, most preferably 1 to 20 nm.

## Revendications

1. Procédé de préparation d'une dispersion contenant des nanoparticules d'argent, en particulier de préparation de ciment osseux ou d'un agent de revêtement pour des implants ou des instruments médicaux ou d'un matériau support antibactérien, comprenant les étapes de :
- mise à disposition d'un sel d'argent,
- mise à disposition d'au moins un stabilisateur,
- mise à disposition d'un agent réducteur,
- mise à disposition d'un solvant organique polymérisable,
- préparation d'une solution de sel d'argent, de stabilisateur et d'agent réducteur, qui contient de l'eau,
- addition d'une base à la solution,
- addition d'un sel inorganique,
l'addition de la base provoquant la précipitation des nanoparticules d'argent qui se dispersent,
le sel inorganique permettant d'hydrater l'eau restant en solution et de former une phase aqueuse, alors que les nanoparticules d'argent restent principalement dans le solvant organique polymérisable compte tenu du stabilisateur ajouté,
- séparation de la phase aqueuse se formant après l'addition du sel inorganique et du mélange comprenant les nanoparticules d'argent et les stabilisateurs, si bien qu'il reste un solvant organique avec des nanoparticules d'argent.

2. Procédé de préparation d'une dispersion contenant des nanoparticules d'argent selon la revendication précédente, **caractérisé en ce qu'**au moins un additif de réticulation et de dispersion est ajouté au mélange comprenant des nanoparticules d'argent et des stabilisateurs.

3. Procédé de préparation d'une dispersion contenant des nanoparticules d'argent selon l'une des revendications précédentes, **caractérisé en ce que** l'au moins un stabilisateur est choisi dans le groupe constitué de l'ester d'acide polyoxyéthylène-monoalkylique, l'ester d'acide polyoxypropylène-monoalkylique, l'ester d'acide polyoxyéthylène-dialkylique, l'ester d'acide polyoxypropylène-dialkylique, l'ester d'acide polyoxyéthylène-trialkylique, l'ester d'acide polyoxypropylène-trialkylique et de leurs mélanges.

4. Procédé de préparation d'une dispersion contenant des nanoparticules d'argent selon l'une des revendications précédentes, **caractérisé en ce qu'**un acrylate, notamment un méthacrylate de méthyle, est utilisé comme solvant organique polymérisable.

5. Procédé de préparation d'une dispersion contenant des nanoparticules d'argent selon l'une des revendications précédentes, **caractérisé en ce que** l'addition de la base s'effectue en continu, notamment pendant une période de 5 à 48 h, de sorte que le pH de la formulation se situe entre 0 et 6.

6. Procédé de préparation d'une dispersion contenant des nanoparticules d'argent selon l'une des revendications précédentes, **caractérisé en ce que** la phase aqueuse est séparée du mélange contenant des nanoparticules d'argent et des stabilisateurs par décantation.

7. Procédé selon l'une des revendications précédentes, dans lequel l'agent réducteur réagit avec des ions argent du sel d'argent en formant de l'argent élémentaire et des produits réactionnels sinon principalement gazeux et/ou dans lequel de l'hydrazine hydratée est mise en oeuvre comme agent réducteur et/ou dans lequel une base ayant une valeur pKb dans la plage de -2 à 10,5, de préférence une valeur pKb de 1,5 à 9,1, particulièrement préférentiellement une valeur pKb dans la plage de 3,5 à 7,5, est mise en oeuvre comme base et/ou dans lequel de l'ammoniac, de l'hydrogéno-carbonate de potassium ou de l'hydroxyde de sodium est mis en oeuvre comme base et/ou dans lequel l'addition de la base s'effectue en continu pendant une période de 9 à 30 h et/ou dans lequel le sel inorganique comprend au moins un cation de la période 3 ou de la période 4 du système périodique des éléments et au moins un élément du cinquième groupe principal du système périodique des éléments comme composant de l'anion et/ou dans lequel le sel inorganique comprend de l'azote comme composant de l'anion et/ou dans lequel l'additif de réticulation et de dispersion est un éthoxylate d'alkylphénol, un polyester aminofonctionnel, une substance phosphorée, ou un mélange de ces composés et/ou dans lequel l'additif de réticulation et de dispersion est un phosphate organiquement modifié, un phosphonate, un composé polyphosphoré, un alkylphosphonate, un composé phosphoré avec des ligands organiques mélangés, un oligomère ou un polymère avec des ligands phosphatés.

8. Dispersion contenant des nanoparticules d'argent pouvant être préparée par un procédé selon l'une des revendications précédentes, comprenant des nanoparticules d'argent,
au moins un stabilisateur choisi dans le groupe constitué de l'ester d'acide polyoxyéthylène-monoalkylique, l'ester d'acide polyoxypropylène-monoalkylique, l'ester d'acide polyoxyéthylène-dialkylique, l'ester d'acide polyoxypropylène-dialkylique, l'ester d'acide polyoxyéthylène-trialkylique, l'ester d'acide polyoxypropylène-trialkylique et de leurs mélanges,
et au moins un tensioactif non ionique comme additif de réticulation et de dispersion,
les nanoparticules d'argent étant dispersées dans un monomère, un prépolymère ou un polymère liquides,
et les nanoparticules d'argent étant enveloppées d'au moins un stabilisateur et de l'additif de réticulation et de dispersion.

9. Dispersion contenant des nanoparticules d'argent selon la revendication 8, **caractérisée en ce que** l'additif de réticulation et de dispersion est un phosphate organiquement modifié, un phosphonate, un composé polyphosphoré, un alkylphosphonate, un composé phosphoré avec des ligands organiques mélangés, un oligomère ou un polymère avec des ligands phosphatés.

10. Dispersion contenant des nanoparticules d'argent selon l'une des revendications précédentes 8 ou 9, **caractérisée en ce que** la taille moyenne de particule des nanoparticules est comprise entre 5 et 50, de préférence entre 10 et 20 nm et/ou **en ce qu'**au moins 90, de préférence au moins 99 % des nanoparticules d'argent sont inférieures à 50, de préférence inférieures à 20 nm, et/ou **en ce que** les nanoparticules d'argent présentent une forme essentiellement sphérique et/ou **en ce que** la proportion de nanoparticules d'argent est comprise entre 0,5 et 5, de préférence entre 1 et 3 % en poids et/ou **en ce que** le polymère comprend un acrylate ou un précurseur d'acrylate, notamment un méthacrylate de méthyle.

11. Utilisation d'une dispersion contenant des nanoparticules d'argent selon l'une des revendications précédentes 8 à 10 comme monomère, en particulier pour du ciment osseux, une solution de revêtement ou comme additif pour les matériaux polymères, notamment les implants artificiels.

12. Utilisation d'une dispersion contenant des nanoparticules d'argent comprenant des nanoparticules d'argent selon la revendication 11, au moins deux stabilisateurs étant présents dans le mélange contenant des nanoparticules d'argent, lesdits stabilisateurs étant choisis dans le groupe constitué de l'ester d'acide polyoxyéthylène-monoalkylique, l'ester d'acide polyoxypropylène-monoalkylique, l'ester d'acide polyoxyéthylène-dialkylique, l'ester d'acide polyoxypropylène-dialkylique, l'ester d'acide polyoxyéthylène-trialkylique et l'ester d'acide polyoxypropylène-trialkylique, et le rapport quantitatif des nanoparticules d'argent sur le stabilisateur étant compris dans la plage de 10:2 à 10:50, pour la préparation de ciment osseux ou d'un agent de revêtement pour des implants et/ou des instruments médicaux ou d'un matériau support antibactérien.

13. Utilisation selon la revendication 12 précédente, les stabilisateurs étant choisis dans le groupe constitué du monolaurate de polyoxyéthylène-sorbitane, du monopalmitate de polyoxyéthylène-sorbitane, du monostéarate de polyoxy-éthylène-sorbitane, du monooléate de polyoxyéthylène-sorbitane, du tristéarate de polyoxyéthylène-sorbitane, du trioléate de polyoxyéthylène-glycéryle, du monolaurate de polyoxyéthylène-glycéryle, du monooléate de polyoxyéthylène-glycéryle, du monostéarate de polyoxy-éthylène-glycéryle, du monoricinoléate de polyéthylène-glycéryle, de l'huile de ricin, de l'huile de ricin hydrogénée, de l'huile de soja et de leurs mélanges et/ou les stabilisateurs étant présents en un rapport quantitatif compris dans la plage de 1:1 à 2:1 et/ou les nanoparticules d'argent étant présentes en une taille de particules de 1 à 100 nm, de préférence 1 à 50 nm, particulièrement de préférence de 1 à 20 nm.
